# NEUE EUROPÄISCHE PATENTSCHRIFT

(11) **EP 3 194 927 B2**
(45) Veröffentlichungstag und Bekanntmachung des Hinweises auf die Entscheidung über den Einspruch: **18.08.2021**
(45) Hinweis auf die Patenterteilung: 27.02.2019
(21) Anmeldenummer: 15813001.3
(22) Anmeldetag: 10.12.2015
(51) Int. Cl.: G01N 3/40, G01N 33/15

(54) **BRUCHKAMMER ZUM MESSEN EINER HÄRTE EINES PRÜFLINGS**
BREAKING CHAMBER FOR MEASURING A HARDNESS OF A TEST SUBJECT
CHAMBRE DE RUPTURE POUR LA MESURE DE LA DURETÉ D'UNE ÉPROUVETTE

(30) Priorität: 08.09.2015 DE 102015115043
(43) Veröffentlichungstag der Anmeldung: 26.07.2017
(73) Patentinhaber: Kraemer, Thilo, 64291 Darmstadt (DE)
(72) Erfinder: KRAEMER, Thilo, 64291 Darmstadt (DE); JEZIERSKI, Thomas, 63500 Seligenstadt (DE)
(74) Vertreter: Hamel, Armin
(86) Internationale Anmeldenummer: PCT/EP2015/079257
(87) Internationale Veröffentlichungsnummer: WO 2017/041866

(56) Entgegenhaltungen:
- EP-A1- 1 290 438
- EP-B1- 1 290 438
- WO-A1-2013/083308
- WO-A1-2013/083308
- WO-A2-98/19945
- WO-A2-2013/061223
- WO-A2-2013/061223
- DE-A1- 3 711 827
- DE-A1- 19 619 115
- DE-A1- 19 619 115
- DE-A1-102007 036 658
- DE-A1-102007 036 658
- DE-B3-102013 113 126
- DE-B3-102013 113 126
- DE-B3-102015 114 600
- DE-U1- 20 010 461
- DE-U1-202005 010 575
- DE-U1-202014 101 160
- DE-U1-202014 101 160
- DE-U1-202014 101 711
- DE-U1-202014 101 711
- DE-U1-202015 101 878
- DE-U1-202015 101 878
- US-A- 4 393 717
- US-A- 4 393 717
- US-A- 4 555 028
- US-A- 4 884 463
- US-A- 4 884 463
- US-A- 5 099 682
- US-A1- 2013 176 553
- CONRADS, GEORG: "Hochpräziser Testtisch für vertikale und horizontale Vibration", BWI-BetonWerk International, 2010, page 56,
- Anonymous: "Gerichtete Vibration. Kosten sparen bei höchster Verdichtungsqualität", BOMAG Fayat Group, March 2009 (2009-03), pages 1-8,
- anonymous: "Artikelliste Gummi-Metall-Puffer zylindrisch", Lohmann Gummi-Metall-Verbindungen, 1 August 2015 (2015-08-01), page 64pp,

## Beschreibung

Die Erfindung betrifft eine Bruchkammer zum Messen einer Härte eines Prüflings umfassend eine Festbacke sowie eine ihr gegenüberliegende Pressbacke, wobei die Bruchkammer einen Boden aufweist, der durch einen Teil eines Plattenelements gebildet wird. Ferner betrifft die Erfindung Verfahren zum Messen einer Härte eines Prüflings in dieser Bruchkammer.

Aus DE 200 10 461 U1 betrifft ein Verfahren sowie eine Vorrichtung zum Ausrichten und Verschieben eines Prüflings, wie Tabletten, Pillen, Dragees oder Tablets, welcher wenigstens auf einer der Hauptoberflächen bombiert oder gerundet und somit aus einer Ruhelage heraus zur Ausführung von Kippschwingungen im Stande ist, zur Durchführung eines weiteren Prozessschrittes, wie Härtetest, mit dem Prüfling, unter Zuhilfenahme von zwei sich gegenüberstehenden und relativ zueinander beweglichen Backen, von denen der bewegliche Pressbacken den Prüfling auf einer Führungsbahn auf den feststehenden Gegenbacken bis zum mechanischen Kontakt des Prüflings mit dem Gegenbacken vorschiebt, um den weiteren Prozessschritt mit dem Prüfling einzuleiten, dadurch gekennzeichnet, dass unter Mitnahme des Prüflings die Vorschubbewegung des beweglichen Pressbackens in Richtung des feststehenden Gegenbackens fortlaufend unterbrochen und der Pressbacken zurückgezogen und vorgeschoben wird, wobei die Vorschub- und Rückschubbewegung des Pressbackens klein ist in Bezug auf seinen gesamten Vorschubweg bezüglich des Gegenbackens, insgesamt jedoch der Pressbacken auf den Gegenbacken unter Mitnahme des Prüflings um jeweils die Meine Vorschubstrecke und Lösen von dem Prüfling mittels Rückschub zufährt, so dass der Prüfling in seine Ruhelage zurückschwingt und danach der Pressbacken wieder zum Schub in Richtung des Gegenbackens ansetzt, bis der Prüfling den Gegenbacken erreicht hat oder der feststehende Backen und/oder die Führungsbahn wird während der Vorschubbewegung des beweglichen Pressbackens in Richtung des feststehenden Gegenbackens in Schwingungen versetzt, welche sich dem Prüfling aufprägen, so dass derselbe während der Vorschubbewegung des Pressbackens auch eine Kippschwingung und/oder Vibrationsschwingung durchführt, die den Prüfling fortwährend in die Ruhelage zurück schwingen oder um dieselbe schwingen lässt, bis der Prüfling den Gegenbacken erreicht hat und die Schwingung des Prüflings durch den feststehenden Gegenbacken angehalten wird.

Des Weiteren ist aus WO 2013 061 223 A2 ein Tablettenprüfgerät zur Prüfung von Tabletten mit mindestens einer Tablettenprüfstation, die zur Durchführung mindestens eines Testverfahrens geeignet ist, bekannt. Das Tablettenprüfgerät umfasst vorzugsweise mindestens eine zur Aufnahme und gegebenenfalls auch zur Zerkleinerung der Tabletten geeignete Vorrichtung sowie mindestens eine Vorrichtung zum Positionieren. Die Vorrichtung zum Positionieren der Tabletten umfasst mindestens eine bewegbare Positionierfläche, vorzugsweise eine Klappe, und mindestens eine weitere Fläche, die zum Positionieren der Tabletten mit dieser Positionierfläche zusammenwirkt.

Aufgabe der vorliegenden Erfindung ist es, eine Bruchkammer sowie ein Verfahren bereitzustellen, mit der auch Prüflinge mit komplexen Strukturen schnell und exakt positioniert und während der Härtemessung in einer Bruchachse gehalten werden können.

Diese Aufgabe wird durch die Merkmale der Patentansprüche 1, 7 und 9 gelöst.
[A01] Die Erfindung betrifft somit eine Bruchkammer zum Messen einer Härte eines Prüflings, in der eine Festbacke sowie eine ihr gegenüberliegende Pressbacke angeordnet ist. In der Bruchkammer ist ein Boden vorgesehen, auf dem ein Prüfling angeordnet werden kann. Bei diesen Prüflingen handelt es sich vorzugsweise um Tabletten, beispielsweise um Oblongs. Der Boden wird durch mindestens einen Teil eines Plattenelements gebildet. Ist das Plattenelement größer als der Boden, so wird der Boden nur von einem Teil des Plattenelements gebildet. Es ist aber auch denkbar, dass das Plattenelement exakt die Größe des Bodens hat und somit der Boden durch das gesamte Plattenelement gebildet wird. Das Plattenelement ist mit mindestens einem Antrieb verbunden, der das Plattenelement in eine erste Schwingung sowie eine zweite gerichtete Schwingung versetzen kann, wobei die erste gerichtete Schwingung der zweiten gerichteten Schwingung entgegengesetzt ist. Das Plattenelement wird somit in Vibration versetzt, womit es sich bei dem Plattenelement um ein Vibrationsplattenelement handelt. Wird das Vibrationsplattenelement in Vibration versetzt, so wird der Prüfling in die gewünschte Position bewegt und dort exakt positioniert. Vorteilhaft ist dabei, dass der Prüfling auch sehr komplexe Strukturen aufweisen kann. Ein weiterer Vorteil besteht darin, dass im Falle von Oblongs diese, selbst dann, wenn sie auf einer Schmalseite, d.h. mit einer Kante auf dem Boden, liegen, bei der Vibration des Vibrationsplattenelements direkt umgeworfen werden und so auf der Längsseite angeordnet sind. Dadurch ist es nicht erforderlich, eine zusätzliche Vorrichtung vorzusehen, mit der die Oblongs umgeworfen werden, damit die Härtemessung gemäß dem Europäischen bzw. dem US-amerikanischen Arzneibuch durchgeführt werden kann. Vorteilhaft ist ferner, dass durch die Vibration des Plattenelements auch der Staub oder Krümel des Prüflings aus der Bruchkammer transportiert wird. Das Plattenelement kann aber auch mit zwei Antrieben verbunden sein. Vorteilhaft ist dabei, dass durch Einschalten des ersten Antriebs der Prüfling durch die gerichtete Schwingung in eine erste Richtung bewegt wird. Anschließend wird der erste Antrieb abgeschaltet und der zweite Antrieb eingeschaltet, so dass der Prüfling in eine zweite Richtung bewegt wird. Sind unterhalb des Plattenelements zwei Antriebe vorgesehen, so kann zusätzlich zu einem ersten horizontal angeordneten Dämpfungselement ein zweites Dämpfungselement vorgesehen sein. Dabei ist jeweils ein horizontal angeordnetes Dämpfungselement einem Antrieb zugeordnet. Dadurch ist es möglich, die gerichtete Schwingung eines jeden Antriebs und dadurch die Förderwirkung des Prüflings zu beeinflussen.
[A02] Bevorzugt ist der Antrieb ein Unwuchtmotor. Mit diesem Unwuchtmotor ist das Plattenelement in eine erste gerichtete Schwingung versetzbar. Durch diese gerichtete Schwingung wird das Plattenelement in Vibration versetzt und der auf dem Boden liegende Prüfling in eine erste definierte Richtung bewegt. Wird der Unwuchtmotor umgepolt, so wird das Plattenelement in eine der ersten Schwingung entgegengesetzten Schwingung versetzt. Dadurch wird der Prüfling in eine der ersten Richtung entgegengesetzten zweiten Richtung bewegt. Dadurch ist es möglich, den Prüfling exakt in der Bruchkammer zu positionieren und auch in dieser Position zu halten als auch den Prüfling innerhalb der Bruchkammer umzupositionieren. Vorteilhaft bei diesem Unwuchtmotor ist ferner, dass dieser sehr preiswert ist. So kann dieser Unwuchtmotor beispielsweise ein Vibrator eines Mobiltelefons sein.
[A03] Vorzugsweise ist unterhalb des Plattenelements mindestens ein Dämpfungselement vorgesehen, das zwischen einem ersten Klemmelement und einem zweiten Klemmelement angeordnet ist, wobei das erste Klemmelement mit dem Plattenelement verbunden ist. Vorteilhaft bei dieser Anordnung ist, dass das mindestens eine Dämpfungselement zwischen den beiden Klemmelementen fixiert ist. Die beiden Klemmelemente sowie die Dämpfungselemente bilden damit eine Dämpfereinheit. Ist das mindestens eine Dämpferelement schräg oder vertikal angeordnet, so dämpft dieses Dämpfungselement die Schwingung in vertikaler Richtung. Es handelt sich somit um eine vertikale Dämpfereinheit. Diese Dämpfereinheit ist über das erste Klemmelement mit dem Plattenelement verbunden ist, wird die Vibration des Plattenelements in vertikaler Richtung gedämpft. Neben den schräg oder vertikal angeordneten Dämpfungselementen können auch horizontal angeordnete Dämpfungselemente vorgesehen sein, mit dem die Vibration gezielt abgebremst werden kann. Dadurch wird der Prüfling zwar weiterhin schnell in die gewünschte Richtung transportiert, jedoch verhindert das Dämpfungselement, dass der Prüfling während der Bewegung auf dem Boden herum hüpft. Der Prüfling gleitet somit praktisch auf dem Boden in die vordefinierte Richtung.
[A04] In einer bevorzugten Ausführungsform sind zwischen dem ersten Klemmelement und dem zweiten Klemmelement mindestens drei Dämpfungselemente angeordnet. Die Klemmelemente können dabei als Klemmringe ausgebildet sein. Vorzugsweise sind diese mindestens drei Dämpfungselemente zwischen den beiden Klemmelementen symmetrisch angeordnet. Bevorzugt werden 3 bis 12 Dämpfungselemente zwischen den beiden Klemmelementen angeordnet. Dadurch erfährt das Plattenelement eine optimale Dämpfung in vertikaler Richtung.
[A05] Bevorzugt enthält das mindestens eine Dämpfungselement ein Elastomer. Dabei ist es auch denkbar, dass das Dämpfungselement bzw. die Dämpfungselemente aus jeweils verschiedenen Elastomeren bestehen. Von Vorteil ist dabei, dass diese Elastomere gute Dämpfungseigenschaften haben und recht preiswert in der Anschaffung sind. Besonders bevorzugt wird als Elastomer Naturkautschuk oder Silikonkautschuk verwendet, weil Naturkautschuk bzw. Silikonkautschuk preiswert in der Anschaffung ist und diese Elastomere besonders gute Dämpfungseigenschaften besitzen.
[A06] Besonders bevorzugt ist das mindestens eine Dämpfungselement ein Gummi-Metall-Dämpfer, weil dieser Dämpfer die Vibration des Plattenelements besonders gut abdämpft.
[A07] Die Erfindung betrifft auch ein erstes Verfahren zum Durchführen einer Härtemessung an einem Prüfling in dieser Bruchkammer, das folgende aufeinanderfolgende Schritte umfasst:
   1. Es wird ein Prüfling in der Bruchkammer bereitgestellt, wobei der Prüfling mit einer ersten Längsseite bereits an dem ersten Widerlager anliegt;
   2. es wird die Pressbacke in Richtung der Festbacke bewegt, bis der Prüfling zwischen der Pressbacke und der Festbacke eingeklemmt ist;
   3. es wird die Länge des Prüflings vermessen;
   4. die Pressbacke wird in eine Ausgangsposition zurückgefahren;
   5. der Antrieb wird eingeschaltet und das Plattenelement dadurch in eine erste gerichtete Schwingung versetzt, die der ersten gerichteten Schwingung entgegengesetzt ist, wodurch der Prüfling gedreht wird, bis er mit seiner zweiten Längsseite an der Festbacke anliegt;
   6. die Pressbacke wird in Richtung der Festbacke geschoben, bis der Prüfling zwischen der Festbacke und der Pressbacke eingeklemmt ist;
   7. es wird die Breite des Prüflings vermessen;
   8. die Pressbacke wird in ihre Ausgangsposition zurückgefahren;
   9. der Antrieb wird umgepolt und das Plattenelement in die zweite gerichtete Schwingung versetzt, wodurch der Prüfling gedreht wird, so dass dieser mit seiner ersten Längsseite wieder an dem Widerlager angeordnet ist;
   10.die Pressbacke wird wieder in Richtung der Festbacke bewegt und es wird solange Kraft auf den Prüfling ausgeübt, bis dieser zerbricht.

Nach Durchführung des Bruchtests können die Reste des Prüflings einfach aus der Bruchkammer entfernt werden. Eine Vorrichtung zum Reinigen, zum Beispiel eine Bürstenvorrichtung, ist dazu nicht erforderlich. Die Bruchkammer ist damit selbstreinigend.

Soll die Breite des Prüflings nicht vermessen werden, so kann auf die Schritte 5 bis 9 verzichtet werden.

Vorzugsweise wird auch bei den Schritten 3 bis 5 das Plattenelement weiterhin der ersten Schwingung ausgesetzt, womit der Prüfling in der entsprechenden Position gehalten wird.

Vorteilhaft bei diesem Verfahren ist, dass der Prüfling schnell und einfach in der Bruchkammer positioniert und auch in einer bestimmten Position gehalten werden kann, was dadurch erreicht wird, dass das Plattenelement in Schwingung versetzt werden kann.

Des Weiteren betrifft die Erfindung ein zweites Verfahren zum Messen einer Härte eines Prüflings mit den folgenden aufeinanderfolgenden Schritten:
1. Es wird ein Prüfling in der Bruchkammer bereitgestellt, wobei der Prüfling mit einer ersten Längsseite bereits an dem ersten Widerlager anliegt;
2. der Antrieb wird eingeschaltet und das Plattenelement in eine erste gerichtete Schwingung versetzt, wodurch der Prüfling in der Bruchkammer derart umpositioniert wird, dass der Winkel α der ersten Längsseite des Prüflings in Bezug auf das erste Widerlager etwa 35° bis 55° und vorzugsweise etwa 45° beträgt. Die Positionierung erfolgt dadurch, dass ein Ende des Prüflings, das näher zur Pressbacke angeordnet ist, von dem Widerlager wegbewegt wird, wobei das andere Ende weiterhin an dem Widerlager angeordnet bleibt;
3. die Pressbacke wird in Richtung der Festbacke bewegt, bis der Prüfling mit seiner zweiten Längsseite an der Festbacke anliegt und zwischen der Festbacke und der Pressbacke eingeklemmt ist;
4. es wird die Breite des Prüflings vermessen;
5. anschließend wird die Pressbacke wieder in die Ausgangsposition zurückbewegt;
6. sodann wird der Antrieb umgepolt und das Plattenelement in eine zweite gerichtete Schwingung versetzt, wodurch der Prüfling in der Bruchkammer derart umpositioniert wird, dass er mit seiner ersten Längsseite wieder an dem Widerlager anliegt;
7. die Pressbacke wird in Richtung der Festbacke bewegt, bis der Prüfling zwischen der Pressbacke und der Festbacke eingeklemmt ist;
8. es wird die Länge des Prüflings vermessen;
9. es wird solange Kraft auf den Prüfling ausgeübt, bis dieser zerbricht.

Vorzugsweise wird der Antrieb bei der Durchführung des Schritts 7 nicht ausgeschaltet, so dass der Prüfling in dieser Position einfach dadurch gehalten wird, weil dieser Prüfling durch die gerichtete Schwingung des Plattenelements gegen das Widerlager geschoben wird. Nach Durchführung des Bruchtests werden die Reste des Prüflings aus der Bruchkammer entfernt, ohne dass zusätzliche Reinigungsvorrichtungen erforderlich wären. Damit ist die Bruchkammer selbstreinigend. So werden größere Reste des zerbrochenen Prüflings mittels einer Transportvorrichtung aus der Bruchkammer entfernt. Daneben können kleine aber auch größere Reste und insbesondere Staub durch die Vibration des Plattenelements aus der Bruchkammer transportiert bewegt werden.

Soll die Breitenmessung nicht durchgeführt werden, so kann auf die Schritte 2 bis 6 verzichtet werden.

Auch mit diesem Verfahren kann der Prüfling schnell und einfach in der Bruchkammer positioniert und auch in einer bestimmten Position gehalten werden, was durch den Antrieb ermöglicht wird, der das Plattenelement in Vibration versetzt.

[A9] Die Erfindung betrifft zudem ein drittes Härtemessverfahren mit den folgenden aufeinanderfolgenden Schritten:
1. Es wird ein Prüfling in der Bruchkammer bereitgestellt, wobei der Prüfling in einer Ausrichtung befindet, die parallel zu der schrägen Seite des Widerlagers angeordnet ist;
2. es wird die Pressbacke in Richtung der Festbacke bewegt, bis der Prüfling mit einer ersten Längsseite an der Pressbacke anliegt, wobei der Prüfling zudem zwischen der Pressbacke und der Festbacke eingeklemmt ist;
3. es wird die Breite des Prüflings vermessen;
4. die Pressbacke wird in eine Ausgangsposition zurückgefahren;
5. der Antrieb wird eingeschaltet und das Plattenelement in eine erste gerichtete Schwingung versetzt, wodurch der Prüfling gedreht wird, bis der Prüfling mit seiner zweiten Längsseite an dem Widerlager angeordnet ist;
6. die Pressbacke wird in Richtung der Festbacke bewegt, bis der Prüfling zwischen der Festbacke und der Pressbacke eingeklemmt ist;
7. es wird die Länge des Prüflings vermessen;
8. die Pressbacke wird weiter in Richtung der Festbacke bewegt und es wird solange Kraft auf den Prüfling ausgeübt, bis dieser zerbricht.

Nach Durchführung des Bruchtests werden die Reste des zerbrochenen Prüflings aus der Bruchkammer entfernt. Von Vorteil ist somit, dass die Bruchkammer selbstreinigend ist.

Es versteht sich, dass auf die Breitenmessung und damit auf Schritt 3 verzichtet werden kann.

Auch mit diesem Verfahren kann der Prüfling schnell und einfach in der Bruchkammer positioniert und auch in einer bestimmten Position gehalten werden, was wiederum durch den Antrieb ermöglicht wird, der das Plattenelement in Schwingungen versetzen kann.

Ausführungsbeispiele der Erfindung werden im Folgenden anhand von Figuren näher erläutert. Es zeigen:
- Figur 1: eine schematische Darstellung eines Ausschnitts eines Prüfgeräts;
- Figur 2: einen Schnitt A-A durch den in Figur 1 gezeigten Ausschnitt des Prüfgeräts;
- Figur 3: eine perspektivische Ansicht eines Plattenelements und einem daran angeordneten unteren Abschnitt;
- Figuren 4a bis 4d: ein erstes Verfahren zum Messen einer Härte eines Prüflings in einer Bruchkammer;
- Figuren 5a bis 5d: ein zweites Verfahren zum Messen einer Härte eines Prüflings in einer Bruchkammer;
- Figuren 6a bis 6d: ein drittes Verfahren zum Messen einer Härte eines Prüflings in einer Bruchkammer;
- Figur 7: den unteren Abschnitt des in Figur 3 gezeigten Plattenelements ohne Halter und Gehäuse;
- Figur 8: eine erste Variante eines unteren Abschnitts des in Figur 7 gezeigten Plattenelements;
- Figur 9: eine zweite Variante eines unteren Abschnitts eines Plattenelements;
- Figur 10: eine dritte Variante eines unteren Abschnitts eines Plattenelements;
- Figur 11: eine vierte Variante eines unteren Abschnitts eines Plattenelements;
- Figur 12: eine fünfte Variante eines unteren Abschnitts eines Plattenelements
- Figur 13: eine sechste Variante eines unteren Abschnitts eines Plattenelements.

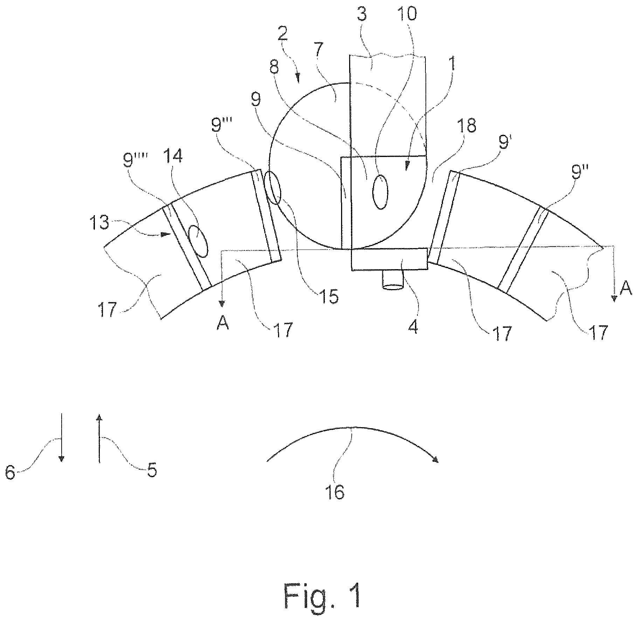

In Figur 1 ist eine schematische Darstellung einer ersten Variante einer Bruchkammer 1 zum Messen einer Härte eines Prüflings 10 gezeigt, wobei die Bruchkammer 1 Teil eines Prüfgeräts 2 ist. Bei diesem Prüfling 10 handelt es sich vorzugsweise um eine Tablette, beispielsweise um einen Oblong. In der Bruchkammer 1 ist eine Festbacke 3 sowie eine ihr gegenüberliegende, bewegliche Pressbacke 4 angeordnet. Diese Pressbacke 4 kann in Richtung der Festbacke 3 hin bewegt oder von dieser fort bewegt werden, was durch den Pfeil 5 bzw. den Pfeil 6 angedeutet ist. Das Prüfgerät 2 weist ein Plattenelement 7 auf, wobei ein Teil des Plattenelements 7 einen Boden 8 der Bruchkammer 1 bildet. In Figur 1 bildet ein Viertel des kreisförmigen Plattenelements 7 den Boden 8 der Bruchkammer 1. Das Plattenelement 7 ist in Figur 1 eine kreisförmige Platte, wobei das Plattenelement 7 auch eine andere Form, zum Beispiel eine ovale, rechteckige oder quadratische Form, aufweisen kann. Dieses Plattenelement 7 ist mit einem mindestens einem Antrieb verbunden, wobei dieser mindestens eine Antrieb unterhalb des Plattenelements 7 oder auch außerhalb der Bruchkammer 1 und damit nicht unterhalb des Plattenelements 7 angeordnet sein kann. Ein Antrieb ist in der Figur 1 allerdings nicht gezeigt. Mittels des Antriebs, beispielsweise eines Unwuchtmotors, kann die Platte in Schwingung versetzt werden, so dass die Platte vibriert. Die Bruchkammer 1 wird zu der einen Seite von dem Widerlager 9 und auf der anderen Seite von einem weiteren Widerlager 9' begrenzt. Diese Widerlager 9 und 9' sowie die weiteren Widerlager 9", 9'" und 9"" gehören zu einer Transportvorrichtung 13, die jedoch nicht im Detail dargestellt ist. Bei der Transportrichtung 13 kann es sich beispielsweise um einen Transportstern oder einen Transportrechen handeln. Da solche Transportvorrichtungen aus dem Stand der Technik bekannt sind, werden diese nicht weiter im Detail beschrieben. Zwischen den Widerlagern 9 und 9' und den Widerlagern 9" und 9'" ist jeweils ein weiterer Prüfling 14, 15 zu sehen, wobei der Prüfling 14 an dem Widerlager 9"" und der Prüfling 15 an dem Widerlager 9'" angeordnet ist. Mit dieser Transportvorrichtung 13 können weitere Prüflinge 14, 15 nacheinander in die Bruchkammer 1 transportiert werden, nachdem an dem Prüfling 10 der Bruchtest durchgeführt wurde. Die Prüflinge 14, 15 werden somit entlang einer Transportebene 17 in Richtung des Pfeils 16 in die Bruchkammer 1 bewegt. Vorzugsweise ist unterhalb des Plattenelements 7 ein Abfallbehälter angeordnet, der jedoch in der Figur 1 nicht zu sehen ist. Wird ein Prüfling, zum Beispiel der Prüfling 10, durch einen Bruchtest zerbrochen, so werden größere Reste durch das Widerlager 9 in Richtung einer Öffnung 18, die zwischen dem Plattenelement 7 und der Transportebene 17 angeordnet ist, transportiert. Kleine Reste und sogar Staub werden automatisch durch die Vibration des Plattenelements 7 aus der Bruchkammer 1 entfernt. Dadurch, dass die Reste eines zerbrochenen Prüflings aber auch Staub allein durch die Vibration des Plattenelements aus der Bruchkammer entfernt werden können, ist die Bruchkammer selbstreinigend. Auf zusätzliche Vorrichtungen zum Entfernen von Resten eines Prüflings, wie zum Beispiel einer Bürste, kann daher verzichtet werden.

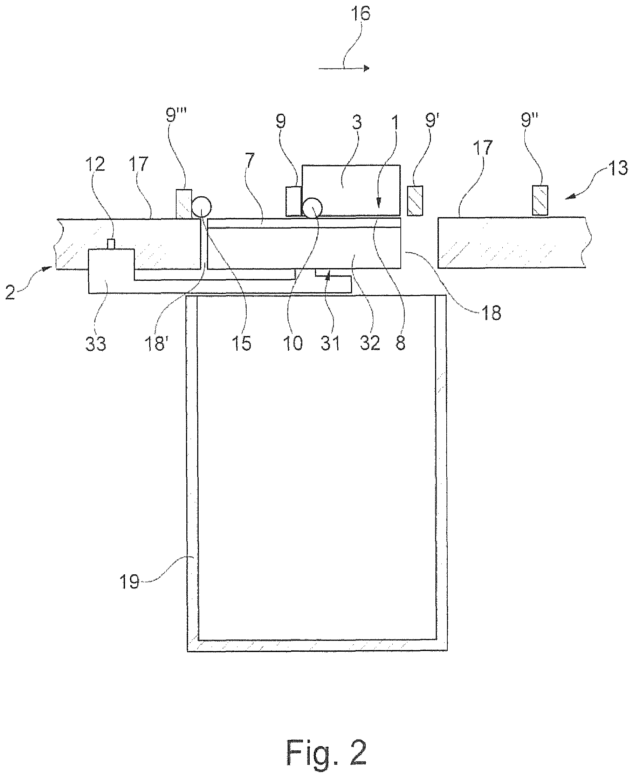

In Figur 2 ist eine schematische Darstellung eines Ausschnitts des Prüfgeräts 2 gemäß Figur 1 gezeigt, wobei ein Schnitt A-A durchgeführt wurde. Dabei ist auch die in der Bruchkammer 1 angeordnete Festbacke 3 zu sehen. Die Pressbacke ist in dieser Ansicht nicht zu sehen. Der Boden 8 der Bruchkammer 1 wird durch einen Teil des Plattenelements 7 gebildet. An dem Plattenelement 7 ist ein unterer Abschnitt 31 angebracht. Auf dem Boden 8 der Bruchkammer 1 liegt der Prüfling 10. Die Bruchkammer 1 wird zu der einen Seite von dem Widerlager 9 und auf der anderen Seite von dem weiteren Widerlager 9' begrenzt. Diese Widerlager 9 und 9' sowie die weiteren Widerlager 9", 9'" und 9"" gehören zu der Transportvorrichtung 13, die jedoch nicht im Detail dargestellt ist. Bei der Transportrichtung 13 kann es sich beispielsweise um einen Transportstern oder einen Transportrechen handeln. Mit dieser Transportvorrichtung 13 können weitere Prüflinge 14, 15 nacheinander in die Bruchkammer 1 transportiert werden, nachdem an dem Prüfling 10 der Bruchtest durchgeführt wurde und die Reste des zerbrochenen Prüflings 10 aus der Bruchkammer 1 entfernt wurden. Die Prüflinge 14, 15 werden somit entlang einer Transportebene 17 in Richtung des Pfeils 16 in die Bruchkammer 1 bewegt. Das Plattenelement 7 mit dem daran angeordneten unteren Abschnitt 31 ist über einen Halter 33 mit dem Prüfgerät 2 derart verbunden, dass die Oberfläche des Plattenelements 7 von der Transportebene 17 entkoppelt ist, wodurch das Plattenelement 7 durch den nicht zu sehenden Antrieb zu Vibrationen angeregt werden kann. Das Plattenelement 7 ist damit schwingbar in dem Prüfgerät 2 aufgehängt. Der Halter 33 weist zumindest ein Verbindungselement auf, wobei in der Figur 2 nur ein Verbindungselement 12 zu sehen ist. Bei diesem mindestens einen Verbindungselement kann es sich zum Beispiel um eine Schraube handeln. Vorzugsweise ist unterhalb des Plattenelements 7 mit dem daran angeordneten unteren Abschnitt 31 ein Abfallbehälter 19 angeordnet. Wird ein Prüfling, zum Beispiel der Prüfling 10, durch einen Bruchtest zerbrochen, so werden größere Reste durch das Widerlager 9 in Richtung einer Öffnung 18, die zwischen dem Plattenelement 7 und der Transportebene 17 angeordnet ist, transportiert. Kleine Reste und auch Staub werden automatisch durch die Vibration des Plattenelements 7 aus der Bruchkammer 1 entfernt, wobei diese Reste durch die Öffnung 18 oder die Öffnung 18' in den Abfallbehälter 19 gelangen.

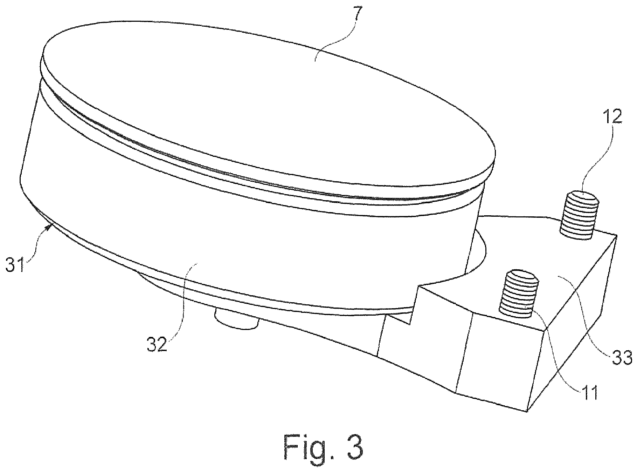

In Figur 3 ist das Plattenelement 7 mit dem daran angeordneten unteren Abschnitt 31 in einer perspektivischen Ansicht gezeigt. Die Bruchkammer 2 ist der Übersicht halber nicht dargestellt. Der untere Abschnitt 31 ist unterhalb des Plattenelements 7 angebracht und mit dem Plattenelement 7 verbunden. Der untere Abschnitt 31 ist von einem Gehäuse 32 umgegeben und sitzt auf einem Halter 33. Mit dem Halter 33 wird der untere Abschnitt 31 mit dem daran angeordneten Plattenelement 7 mittels Verbindungselementen 11, 12 mit dem Prüfgerät (nicht gezeigt) verbunden, so dass die Oberfläche des Plattenelements 7 von einer Transportebene (nicht dargestellt), auf der die Prüflinge zu der Bruchkammer transportiert werden, entkoppelt ist. Dadurch ist es möglich, dass das Plattenelement 7 durch einen Antrieb (nicht zu sehen) in Schwingung versetzt werden kann, wodurch das Plattenelement 7 vibrieren kann.

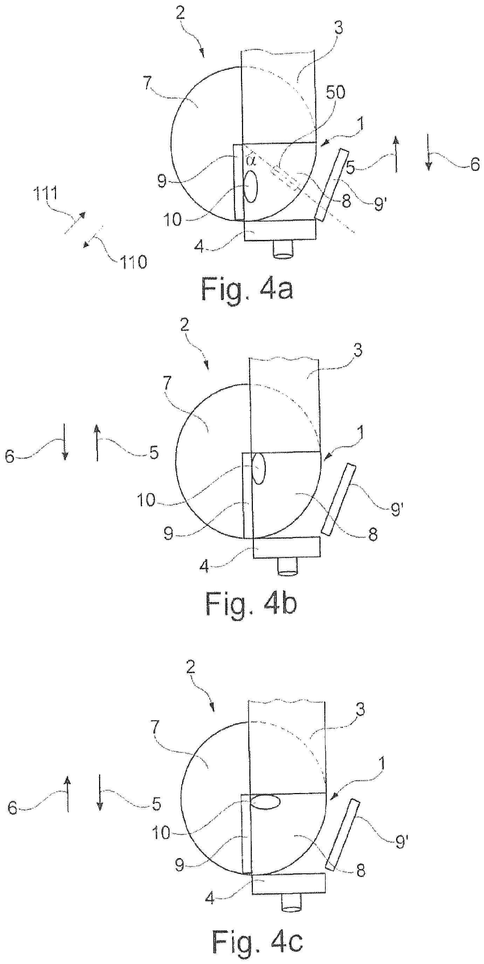

In den Figuren 4a bis 4d ist eine schematische Darstellung der ersten Variante einer Bruchkammer 1 zum Messen einer Härte eines Prüflings gezeigt, wobei die Bruchkammer 1 Teil des nicht weiter dargestellten Prüfgeräts 2 ist. Bei dem Prüfling handelt es sich vorzugsweise um eine Tablette, beispielsweise um einen Oblong. In der Bruchkammer 1 ist die Festbacke 3 sowie die ihr gegenüberliegende, bewegliche Pressbacke 4 angeordnet. Diese Pressbacke 4 kann in Richtung der Festbacke 3 hin bewegt oder von dieser fort bewegt werden, was durch die Pfeile 5 und 6 angedeutet ist. Das Prüfgerät 2 weist das Plattenelement 7 auf, wobei ein Teil des Plattenelements 7 den Boden 8 der Bruchkammer 1 bildet. Dieses Plattenelement 7 ist in Figur 4a eine kreisförmige Platte, wobei das Plattenelement 7 auch eine andere Form, zum Beispiel eine ovale, rechteckige oder quadratische Form, aufweisen kann. Dieses Plattenelement 7 ist mit mindestens einem Antrieb verbunden, wobei dieser mindestens eine Antrieb unterhalb des Plattenelements 7 angeordnet sein kann. Möglich ist aber auch, dass der mindestens eine Antrieb zwar mit dem Plattenelement 7 verbunden ist, jedoch nicht unterhalb dieses Plattenelements 7 angebracht ist. Von Bedeutung ist lediglich, dass der Antrieb derart mit dem Plattenelement 7 verbunden ist, dass durch die Krafteinwirkung des Antriebs das Plattenelement 7 in Vibration versetzen kann. In Figur 4a ist der Antrieb allerdings unterhalb des Plattenelements 7 angeordnet. Er trägt die Bezugszahl 50 und ist durch gestrichelte Linien dargestellt. In Bezug auf das Widerlager 9 ist der Antrieb 50 in einem Winkel α von etwa 35° bis 55° angeordnet, wobei der Antrieb vorzugsweise in einem Winkel von 45° angeordnet ist. Um den Antrieb 50 in einem solchen Winkel anzuordnen, sind der Antrieb 50 und das Plattenelement 7 relativ zueinander bewegbar angeordnet. In Figur 4a ist der Antrieb 50 in einem Winkel α von etwa 45° in Bezug auf das Widerlager 9 angeordnet. In den Figuren 4b bis 4d ist der Antrieb 50 der Übersicht halber nicht mehr gezeigt.

Vorzugsweise handelt es sich bei dem Antrieb 50 um einen Unwuchtmotor, der das Plattenelement 7 in eine erste gerichtete Schwingung versetzen kann. Dieser Antrieb kann beispielsweise in einem Bereich von etwa 1,4 bis 1,8 V arbeiten. Durch Umpolung des Antriebs 50 wird das Plattenelement 7 in eine zweite gerichtete Schwingung versetzt, die der ersten gerichteten Schwingung entgegengesetzt ist. Der auf dem Plattenelement 7 liegende Prüfung 10 wird durch die erste gerichtete Schwingung in eine erste Richtung 110 und nach Umpolung des Antriebs 50 in die zweite gerichtete Richtung 111 bewegt, wobei die erste Bewegungsrichtung 110 des Prüflings 10 der zweiten Bewegungsrichtung 111 entgegengesetzt ist. Die Richtung der entsprechenden gerichteten Schwingungen des Plattenelements 7 entsprechen dabei im Wesentlichen den Bewegungsrichtungen des Prüflings 10. Möglich ist auch, dass zusätzlich noch ein zweiter Antrieb vorgesehen ist, was jedoch in den Figuren 4a bis 4d nicht der Fall ist. Auch dieser zweite Antrieb kann ein Unwuchtmotor sein.

Das erste Widerlager 9, das die Bruchkammer 2 zu einer Seite hin begrenzt, erstreckt sich von der Festbacke 3 in Richtung der Pressbacke 4, wobei das Widerlager 9 im Wesentlichen in einem rechten Winkel zu der Festbacke 3 sowie auch zu der Pressbacke 4 angeordnet ist. Dieses Widerlager 9 ist ein Teil einer Transportvorrichtung, mit der ein Prüfling 10 zur Vermessung in die Bruchkammer 2 transportiert wird. Neben dem Widerlager 9 ist auch das Widerlager 9' der Transportvorrichtung zu sehen. Bei der Transportvorrichtung kann es sich beispielsweise um einen Transportstern oder einen Transportrechen handeln. Da solche Transportvorrichtungen bekannt sind, wir auf eine detaillierte Beschreibung verzichtet.

Der in die Bruchkammer 1 eingebrachte Prüfling 10 liegt dabei auf dem Boden 8, wobei der Prüfling 10 mit einer ersten Längsseite an dem Widerlager 9 angeordnet ist (Figur 4a). Bei diesem Prüfling 10 handelt es sich um einen Oblong.

Anschließend wird die Pressbacke 4 in Richtung des Pfeils 5 bewegt, bis dieser Prüfling 10 mit der Festbacke 3 in Kontakt steht (Figur 4b). Der Prüfling 10 ist dabei zwischen der Festbacke 3 und der Pressbacke 4 eingeklemmt. Es wird sodann die Länge des Prüflings 10 gemessen. Nachdem die Längenmessung erfolgt ist, wird die Pressbacke 4 wieder in eine Ausgangsposition, d.h. in Richtung des Pfeils 6, zurück bewegt (Figur 4b). Dabei ist es möglich, dass während der Längenmessung der mindestens eine Antrieb abgeschaltet bleibt. Es ist aber auch möglich, den Antrieb während der Messung einzuschalten, wobei der Antrieb das Plattenelement 7 in eine erste gerichtete Schwingung versetzt, so dass der Prüfling 10 in Richtung des Pfeils 110 bewegt wird. Dadurch wird der Prüfling 10 zum einen gegen das Widerlager 9 geschoben und zum anderen durch die Pressbacke 4 in Richtung der Pressbacke 3 gedrückt. Dadurch ist es nicht möglich, dass sich der Prüfling 10 bei der Längenmessung plötzlich wegdreht.

Nach der Längenmessung erfolgt eine Umpolung des Antriebs 50, wodurch eine zweite gerichtete Schwingung erzeugt und der Prüfling 10 gedreht wird, bis er die in Figur 4c gezeigte Position erreicht. In dieser Position liegt der Prüfling 10 mit einer Längsachse an der Festbacke 3. Ist diese Position erreicht, so wird die Pressbacke 4 in Richtung der Festbacke 3 bewegt, bis die Pressbacke 4 mit dem Prüfling 10 in Kontakt steht. Ist der Prüfling 10 zwischen der Festbacke 3 und der Pressbacke 4 eingeklemmt, so erfolgt eine Breitenmessung. Nach der Breitenmessung wird die Pressbacke 4 wieder in die Ausgangsposition zurückgefahren. Es versteht sich, dass auch auf die Breitenmessung verzichtet werden kann, womit der Prüfling 10 nicht in die in Figur 4c gezeigte Position bewegt werden müsste.

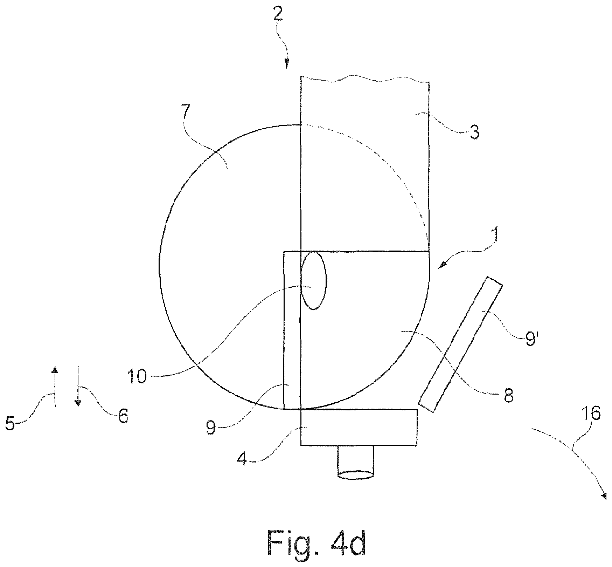

Anschließend wird der Prüfling 10 in die in Figur 4d gezeigte Position bewegt. Dies geschieht dadurch, dass eine Umpolung des Antriebs 50 erfolgt. Dadurch wird der Prüfling 10 durch die vom Antrieb 50 erzeugte gerichtete Schwingung wieder gedreht. Die Bewegung des Prüflings auf dem Plattenelement 7 ist dabei stets linear.

Nachdem der Prüfling 10 die in Figur 4d gezeigte Position eingenommen hat, wird die Pressbacke 4 in Richtung der Festbacke 3, das heißt in Richtung des Pfeils 5, bewegt, bis die Pressbacke 4 mit dem Prüfling 10 in Kontakt steht, wobei der Prüfling 10 in einer Bruchachse ausgerichtet ist. Dabei wird das Plattenelement 7 weiter einer gerichteten Schwingung ausgesetzt, so dass der Prüfling 10 während der Härtemessung in seiner Bruchachse gehalten wird. Dies geschieht dadurch, dass der Prüfling 10 zum einen gegen das Widerlager 9 geschoben und zum anderen durch die Pressbacke 4 in Richtung der Pressbacke 3 (vergleiche Pfeil 5) gedrückt wird. Dadurch ist es nicht möglich, dass sich der Prüfling 10 bei der Härtemessung plötzlich wegdreht. Denkbar ist natürlich auch, dass der Antrieb 50 während Härtemessung ausgeschaltet bleibt.

Es wird sodann der Härtetest durchgeführt, wobei die auf den Prüfling 10 wirkende Kraft solange erhöht wird, bis der Prüfling 10 zerbricht. Die Kraft, die dazu erforderlich ist, wird mittels einer Kraftmessdose ermittelt, die entweder in der Pressbacke 4 oder in der Festbacke 3 untergebracht sein kann. Diese Kraftmessdose ist in den Figuren 4a bis 4d nicht zu sehen.

Nachdem der Prüfling 10 zerbrochen wurde, wird dieser aus der Bruchkammer 2 entfernt. Dies geschieht dadurch, dass größere Stücke des Prüflings 10 durch Bewegung der Transportvorrichtung und damit die Bewegung des Widerlagers 9 in Richtung des Pfeils 16 hinausgeschoben wird. Kleine Reste - darunter auf Staub - des Prüflings 10 werden aus der Bruchkammer 1 hinaus befördert, indem das Plattenelement 7 einer Vibration ausgesetzt wird. Dadurch wandern die kleineren Reste bzw. der Staub in Richtung der Pfeile 110 bzw. 111.

Damit ist in den Figuren 4a bis 4d ein erstes Verfahren zum Messen einer Härte eines Prüflings in einer Bruchkammer dargestellt.

Das Verfahren umfasst folgende Schritte:
1. Es wird ein Prüfling in der Bruchkammer 1 bereitgestellt, wobei der Prüfling mit einer ersten Längsseite bereits an dem ersten Widerlager anliegt (Figur 4a);
2. Es wird die Pressbacke in Richtung der Festbacke bewegt, bis der Prüfling zwischen der Pressbacke und der Festbacke eingeklemmt ist;
3. es wird die Länge des Prüflings vermessen;
4. die Pressbacke wird in eine Ausgangsposition zurückgefahren, so dass die Pressbacke die in Figur 4b gezeigte Position erreicht;
5. der Antrieb wird eingeschaltet und das Plattenelement dadurch in eine zweite gerichtete Schwingung versetzt, wodurch der Prüfling gedreht wird, bis er mit seiner zweiten Längsseite an der Festbacke angeordnet ist (Figur 4c) ;
6. die Pressbacke wird in Richtung der Festbacke geschoben, bis der Prüfling zwischen der Festbacke und der Pressbacke eingeklemmt ist;
7. es wird die Breite des Prüflings vermessen;
8. die Pressbacke wird in ihre Ausgangsposition zurückgefahren;
9. der Antrieb wird umgepolt und das Plattenelement in die erste gerichtete Schwingung versetzt, wodurch der Prüfling gedreht wird, so dass dieser mit seiner ersten Längsseite wieder an dem Widerlager angeordnet ist;
10. die Pressbacke wird wieder in Richtung der Festbacke bewegt und es wird solange Kraft auf den Prüfling ausgeübt, bis dieser zerbricht.

Nach Durchführung des Bruchtests werden alle Reste des zerbrochenen Prüflings 10 aus der Bruchkammer 1 entfernt.

Soll die Breite des Prüflings 10 nicht vermessen werden, so kann auf die Schritte 4 bis 9 verzichtet werden. In diesem Fall ist jedoch möglich, die Pressbacke nur kurz zurückzufahren und dann wieder in Richtung des Prüflings zu bewegen, um anschließend den Bruchtest durchzuführen. Vorzugsweise wird auch bei den Schritten 3 bis 5 sowie bei der Härtemessung (Schritt 10) das Plattenelement 7 weiterhin der ersten Schwingung ausgesetzt, womit der Prüfling 10 in der entsprechenden Position verbleibt und sich nicht wergdreht.

Es ist auch denkbar, dass ein zusätzlicher zweiter Antrieb vorgesehen ist, der ebenfalls eine erste sowie eine zweite gerichtete Schwingung erzeugt, wobei die zweite gerichtete Schwingung der ersten gerichteten Schwingung entgegengesetzt ist. Während der zweite Antrieb in Betrieb ist, bleibt der erste Antrieb abgeschaltet. Vorzugsweise ist jedoch nur ein Antrieb vorgesehen, der mit dem Plattenelement 7 in Verbindung steht.

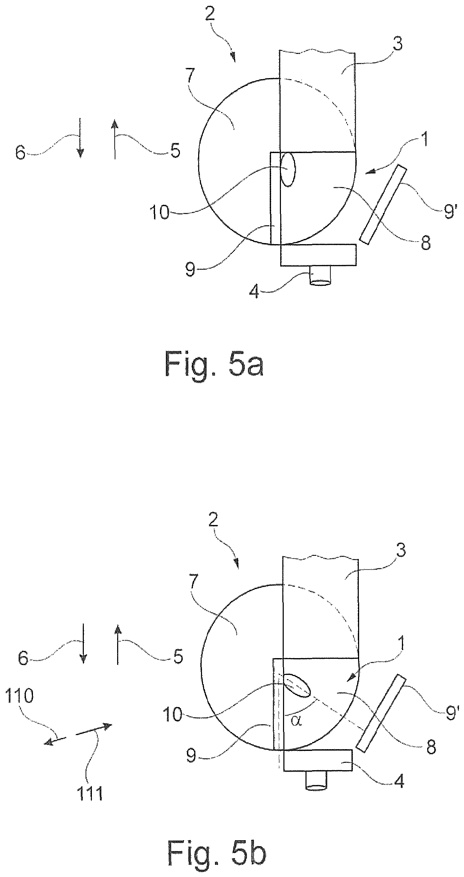

In den Figuren 5a bis 5d ist ein zweites Verfahren zum Messen einer Härte eines Prüflings in einer Bruchkammer gezeigt. Da der Aufbau der Bruchkammer dem Aufbau der in den Figuren 4a bis 4d gezeigten Bruchkammer entspricht, wurden die Bezugszahlen beibehalten.

Die Bruchkammer 1 umfasst wiederum eine Festbacke 3 sowie eine ihr gegenüberliegende Pressbacke 4. Die Bruchkammer 1 besitzt einen Boden 8, der von einem Teil eines Plattenelements 7 gebildet wird. Es ist ein erstes Widerlager 9 vorgesehen, das die Bruchkammer 1 zu einer Seite hin begrenzt und sich von der Festbacke 3 in Richtung der Pressbacke 4 erstreckt, wobei das Widerlager 9 im Wesentlichen in einem rechten Winkel zu der Festbacke 3 sowie auch zu der Pressbacke 4 angeordnet ist. Es ist wiederum ein Antrieb vorgesehen, der mit dem Plattenelement 7 in Kontakt steht, wobei der Antrieb auch unterhalb des Plattenelements 7 angeordnet sein kann, wie dies in Figur 4a dargestellt ist. Dabei kann der Antrieb in Bezug auf das Widerlager 9 wiederum in einem Winkel von 35° bis 55° und vorzugsweise in 45° angeordnet sein. Der Antrieb ist jedoch in den Figuren 5a bis 5d nicht gezeigt. Anstelle eines Antriebs ist es auch möglich, zwei Antriebe vorzusehen. Vorzugsweise steht jedoch nur ein Antrieb mit dem Plattenelement 7 in Verbindung.

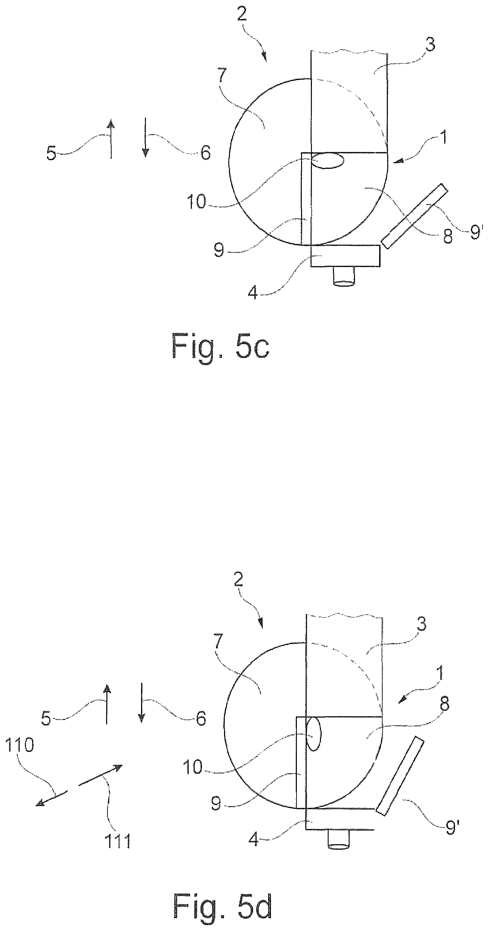

Das Verfahren zum Messen einer Härte eines Prüflings umfasst folgende Schritte:
1. Es wird ein Prüfling 10 in der Bruchkammer 1 bereitgestellt, wobei der Prüfling 10 mit einer ersten Längsseite bereits an dem ersten Widerlager 9 anliegt (Figur 5a);
2. der Antrieb (nicht gezeigt) wird eingeschaltet und das Plattenelement 7 in eine erste gerichtete Schwingung versetzt, wodurch der Prüfling 10 in der Bruchkammer 1 derart umpositioniert wird, dass der Winkel α der ersten Längsseite in Bezug auf das erste Widerlager 9 etwa 35° bis 55° und vorzugsweise etwa 45° beträgt (Figur 5b). Die Positionierung erfolgt dadurch, dass ein Ende des Prüflings 10, das näher zur Pressbacke 4 angeordnet ist, von dem Widerlager 9 wegbewegt wird (Pfeil 111), wobei das andere Ende weiterhin an dem Widerlager 9 angeordnet bleibt;
3. die Pressbacke 4 wird in Richtung der Festbacke 3 bewegt, bis der Prüfling 10 mit seiner zweiten Längsseite an der Festbacke 3 anliegt (Figur 5c) und zwischen der Festbacke 3 und der Pressbacke 4 eingeklemmt ist (nicht gezeigt);
4. es wird die Breite des Prüflings 10 vermessen;
5. anschließend wird die Pressbacke 4 wieder in die Ausgangsposition zurückbewegt;
6. sodann wird der Antrieb umgepolt und das Plattenelement 7 in eine zweite gerichtete Schwingung versetzt, wodurch der Prüfung in der Bruchkammer derart umpositioniert wird, dass er mit seiner ersten Längsseite wieder an dem Widerlager 9 anliegt (Figur 5d);
7. die Pressbacke wird in Richtung der Festbacke bewegt bis der Prüfling 10 zwischen der Festbacke und der Pressbacke eingeklemmt ist;
8. es wird die Länge des Prüflings gemessen
9. die Pressbacke wird weiter in Richtung der Festbacke bewegt und es wird solange Kraft auf den Prüfling ausgeübt, bis dieser zerbricht.

Vorzugsweise wird der Antrieb bei der Durchführung des Schritts 7 und 8 nicht ausgeschaltet, so dass der Prüfling in der in Figur 5d gezeigten Position gehalten wird. Nach Durchführung des Bruchtests werden die Reste des Prüflings aus der Bruchkammer entfernt.

Soll die Breitenmessung nicht durchgeführt werden, so kann auf die Schritte 2 bis 6 verzichtet werden.

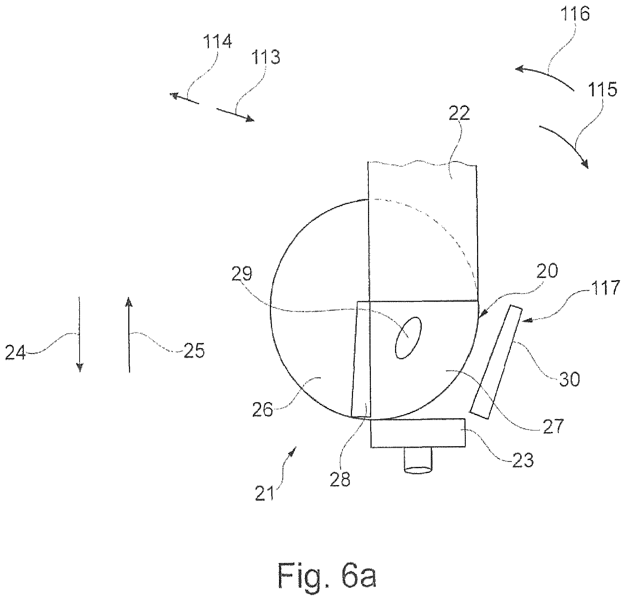

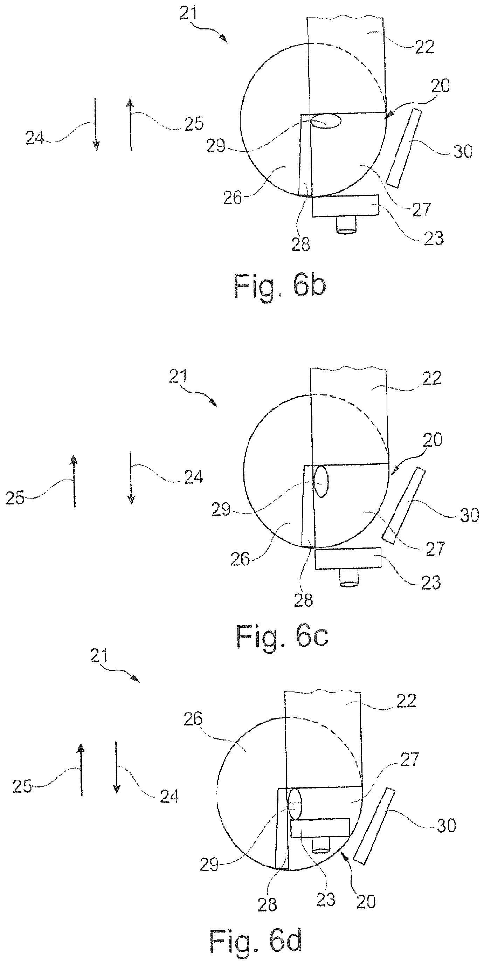

In den Figuren 6a bis 6b ist eine schematische Darstellung einer zweiten Variante der in den Figuren 4a bis 4d gezeigten Bruchkammer dargestellt. Die Bruchkammer 20 ist ebenfalls ein Teil eines Prüfgeräts 21 und weist eine Festbacke 22 sowie eine ihr gegenüberliegende, beweglich angeordnete Pressbacke 23 auf. Diese Pressbacke 23 kann in Richtung der Festbacke 22 hin bewegt oder von dieser fort bewegt werden. Die Richtung dieser Bewegung ist durch die Pfeile 24 und 25 angedeutet. Das Prüfgerät 21 weist ein Plattenelement 26 auf, wobei ein Teil des Plattenelements 26 einen Boden 27 der Bruchkammer 20 bildet. Dieses Plattenelement 26 ist in Figur 6a eine kreisförmige Platte, wobei das Plattenelement 26 auch eine andere Form, zum Beispiel eine ovale, sechseckige, rechteckige oder quadratische Form aufweisen kann. Unterhalb des Plattenelements 26 ist mindestens ein Antrieb vorgesehen, der in dieser Ansicht jedoch nicht dargestellt ist. Mittels dieses mindestens einen Antriebs kann das Plattenelement 26 in Vibration versetzt werden, so dass es sich bei dem Plattenelement 26 um ein Vibrationsplattenelement 26 handelt. Dazu kann der Antrieb das Plattenelement 26 in eine erste gerichtete Schwingung sowie eine zweite gerichtete Schwingung versetzen (Pfeile 113 und 114), wobei die erste gerichtete Schwingung der zweiten gerichteten Schwingung entgegengesetzt ist. Wie auch bei dem Prüfgerät 2 in Figur 1 ist der Antrieb bevorzugt in einem Winkel α von 35° bis 55° und besonders bevorzugt in einem Winkel α von 45° angeordnet. Bevorzugt handelt es sich bei dem Antrieb um einen Unwuchtmotor. Damit das Plattenelement 26 in Vibration versetzt werden kann, ist dieses frei schwingbar in dem Prüfgerät 21 aufgehängt. Das Plattenelement 26 sowie der Antrieb können dabei relativ zueinander bewegt werden, so dass sich der Winkel α einstellen lässt.

Die Bruchkammer 20 umfasst ein erstes Widerlager 28, das die Bruchkammer 20 zu einer Seite hin begrenzt und sich von der Festbacke 22 in Richtung der Pressbacke 23 erstreckt, wobei das Widerlager 28 im Wesentlichen in einem rechten Winkel zu der Festbacke 22 sowie auch zu der Pressbacke 23 angeordnet ist. Wie in den Figuren 6a bis 6d jedoch zu erkennen, weist das Widerlager 28 der Bruchkammer 20 abgewandten Seite eine Schräge auf. Auch dieses Widerlager 28 ist Teil einer Transportvorrichtung 117, mit der ein Prüfling 29 zur Vermessung in die Bruchkammer 20 transportiert wird. Bei der Transportvorrichtung 117 kann es sich beispielsweise um einen Transportstern oder einen Transportrechen handeln.

Der Prüfling 29 wird dabei mittels eines ersten Widerlagers 28 in die Bruchkammer 20 geschoben (vergleiche Pfeil 115), bis dieser Prüfling 29 etwa in der Mitte der Buchtkammer 27 liegt. Anschließend wird die Transportvorrichtung 117 in die entgegengesetzte Richtung (Pfeil 116) bewegt, bis das Widerlager 30 mit seiner schrägen Seite den Prüfling 29 in die Figur 6a gezeigte Position bewegt hat. In dieser Position liegt der Prüfling 29 zwar weiterhin in der Mitte der Bruchkammer 20, jedoch ist dieser Prüfling 29 in einem bestimmten Winkel zu der Pressbacke 23 bzw. der Festbacke 22 angeordnet.

Anschließend wird die Pressbacke 23 in Richtung des Pfeils 25 bewegt und damit der Prüfling 29 in Richtung der Festbacke 22 bewegt, bis der Prüfling 29 mit einer ersten Längsseite an der Festbacke 22 anliegt. Der Prüfling 29 wird dabei schließlich zwischen der Festbacke 22 und der Pressbacke 23 eingeklemmt. In dieser Position wird die Breite des Prüflings 29 gemessen. Danach wird die Pressbacke 23 wieder zurück in die Ausgangsposition (Figur 6b) gefahren, das heißt die Pressbacke 23 wird in Richtung des Pfeils 24 zurück bewegt.

Anschließend wird der Prüfling 29 in die in Figur 6c gezeigte Position bewegt, das heißt der Prüfling wird in Richtung des Pfeils 25 bewegt. Dies geschieht dadurch, dass der Antrieb das Plattenelement 26 in eine erste Schwingung versetzt, so dass der Prüfling 29 in der Bruchkammer 20 umpositioniert wird. Während sich der Prüfling 29 in dieser Position befindet, wird das Plattenelement 26 vorzugsweise weiterhin der ersten gerichteten Schwingung ausgesetzt, so dass der Prüfling 29 in der Bruchachse auch gehalten wird (Figur 6c). Es wird sodann die Pressbacke 23 in Richtung des Prüflings 29 bewegt und der Härtetest durchgeführt. Dazu wird die Kraft der Pressbacke 23 solange erhöht, bis der Prüfling 29 zerbricht. Die Kraft, die erforderlich ist, den Prüfling 29 zu zerbrechen, wird mittels einer Kraftmessdose ermittelt. Diese Kraftmessdose kann entweder in der Pressbacke 23 oder in der Festbacke 22 vorgesehen sein. Diese Kraftmessdose ist in den Figuren 6a bis 6d nicht zu sehen.

Nachdem der Prüfling 29 zerbrochen wurde, wird dieser aus der Bruchkammer 20 entfernt. Zusätzlich wird das Plattenelement 26 einer gerichteten Schwingung ausgesetzt, durch die Staub und kleiner Krümel des Prüflings 29 aus der Bruchkammer 2 hinaus befördert werden.

Das Verfahren umfasst folgende Schritte:
1. Es wird ein Prüfling in der Bruchkammer 1 bereitgestellt, wobei der Prüfling im Wesentlichen parallel zu der schrägen Seite des Widerlagers 28 angeordnet ist (Figur 6a);
2. die Pressbacke wird in Richtung der Festbacke bewegt, bis der Prüfling mit einer ersten Längsseite an der Pressbacke anliegt, wobei der Prüfling zudem zwischen der Pressbacke und der Festbacke eingeklemmt ist;
3. es wird die Breite des Prüflings vermessen;
4. die Pressbacke wird wieder in eine Ausgangsposition zurückgefahren, wobei diese Ausgangsposition in Figur 6b gezeigt ist;
5. der Antrieb wird eingeschaltet und das Plattenelement in eine erste gerichtete Schwingung versetzt, wodurch der Prüfling gedreht wird, bis der Prüfling mit seiner zweiten Längsseite an der Pressbacke angeordnet ist (Figur 6c);
6. die Pressbacke wird in Richtung der Festbacke bewegt, bis der Prüfling zwischen der Festbacke und der Pressbacke eingeklemmt ist;
7. es wird die Länge des Prüflings vermessen;
8. die Pressbacke wird weiter in Richtung der Festbacke bewegt und es wird solange Kraft auf den Prüfling ausgeübt, bis dieser zerbricht.

Nach Durchführung des Bruchtests werden die Reste des Prüflings aus der Bruchkammer entfernt.

Auch hier ist es möglich, die Breitenmessung nicht durchzuführen sondern den Prüfling 29 direkt von der in Figur 6b gezeigten Position durch Drehung in die in Figur 6c gezeigte Position zu bewegen. Allerdings eignet sich dieses Verfahren besonders gut dazu, sehr schnell eine Breitenmessung durchzuführen, weil gerade der zweite Schritt (Schritt 2.) nur sehr wenig Zeit benötigt. Dieses Verfahren ist jedoch nur durchführbar, wenn eine Transportvorrichtung gewählt wird, die Widerlager aufweist, die an einer Seite schräg und damit nicht in einem Winkel von 90° ausgebildet sind. Vorzugsweise ist das Plattenelement 26 nur mit einem Antrieb verbunden. Es ist natürlich auch möglich, zwei Antriebe vorzusehen, die mit dem Plattenelement 26 verbunden sind.

Das Prüfgerät 21 unterscheidet sich somit von dem in den Figuren 4a bis 4d gezeigten Prüfgerät nur durch die Form der Widerlager.

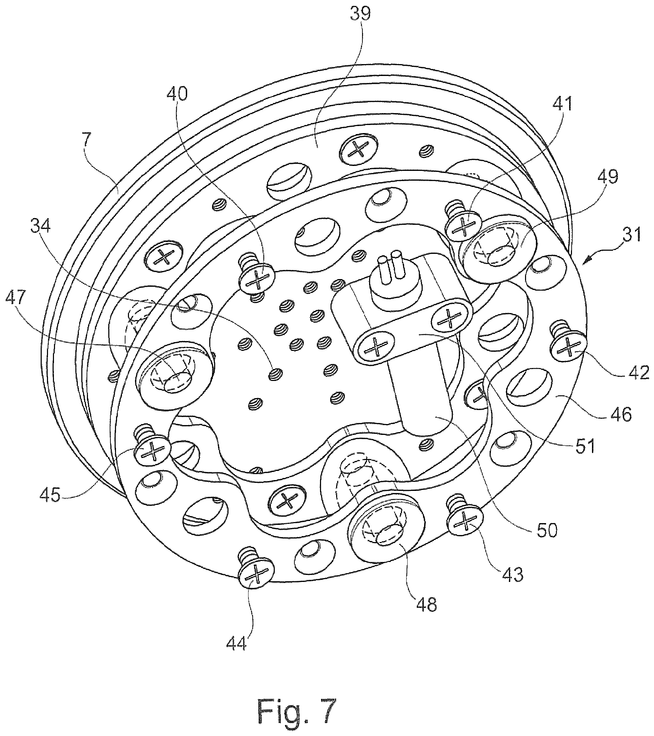

In Figur 7 ist das Plattenelement 7 mit dem daran angeordneten unteren Abschnitt 31 ohne das Gehäuse 32 und ohne den Halter 33 dargestellt. Das Plattenelement 7 mit dem unteren Abschnitt 31 ist dabei gedreht worden, so dass eine Unterseite 34 des Plattenelements 7 zu erkennen ist. An der Unterseite 34 des Plattenelements 7 ist ein erstes Klemmelement 39 angebracht, das über Verbindungsmittel 40 bis 45 mit einem zweiten Klemmelement 46 verbunden ist. Zwischen dem ersten Klemmelement 39 und dem zweiten Klemmelement 46 sind drei vertikal angeordnete Dämpfungselemente 47 bis 49 vorgesehen. Es versteht sich, dass diese Dämpfungselemente 47 bis 49 auch schräg angeordnet sein können (nicht dargestellt), solange diese schräg angeordneten Dämpfungselemente die Schwingung in vertikaler Richtung dämpfen. Die Klemmelemente 39 und 46 sind in diesem Ausführungsbeispiel als Klemmringe ausgebildet.

Im unteren Abschnitt 31 und damit unterhalb des Plattenelements 7 ist der Antrieb 50 vorgesehen, der über ein Halteelement 51 mit dem in Figur 7 nicht dargestellten Halter verbunden ist. Bei diesem Antrieb handelt es sich vorzugsweise um einen Unwuchtmotor. Mit diesem Antrieb 50 ist es möglich, einen auf dem Plattenelement 7 liegenden Prüfling (nicht gezeigt) in zwei verschiedene Richtungen zu bewegen. Dazu wird durch Einschalten des Antriebs 50 eine erste gerichtete Schwingung erzeugt, so dass der Prüfling in eine erste Richtung bewegt wird. Durch Umpolen des Antriebs 50 kann eine zweite gerichtete Schwingung erzeugt werden, wodurch der Prüfling in eine zweite Richtung bewegt wird. Da die zweite gerichtete Schwingung entgegengesetzt zu der ersten gerichteten Schwingungen ist, wird auch der Prüfling durch die zweite gerichtete Schwingung in eine der ersten Bewegungsrichtung entgegengesetzte Richtung bewegt. Die Bewegungen des Prüflings auf dem Plattenelement 7 sind aufgrund der Darstellung in Figur 7 nicht zu sehen. Die gerichteten Schwingungen können dabei nicht nur durch den Antrieb sondern auch durch eine bestimmte Anordnung der Dämpfungselemente gesteuert werden. Auch können die gerichteten Schwingungen dadurch gesteuert werden, dass die Dämpfungselemente die Schwingung unterschiedlich stark abfangen, d.h. diese Schwingungen unterschiedlich stark dämpfen. Dies ist beispielsweise dadurch möglich, dass die Dämpfungselemente aus unterschiedlichen Elastomeren bestehen, die unterschiedliche Dämpfungseigenschaften aufweisen. Die Dämpfungselemente 47 bis 49 können aus einem Elastomer bestehen, wie zum Beispiel aus Naturkautschuk oder aus Silikonkautschuk. Denkbar ist jedoch auch, dass die Dämpfungselemente 47 bis 49 aus einem Gummi-Metall-Dämpfer bestehen.

Es versteht sich, dass der Antrieb 50 auch außerhalb des unteren Abschnitts angeordnet sein kann. In diesem Fall könnte ein Verbindungselement (nicht dargestellt) vorgesehen sein, das mit einem Ende mit dem Antrieb 50 und mit dem anderen Ende mit dem Halteelemente 51 verbunden ist. Durch entsprechende Kraftübertragungselemente ist es möglich, eine von dem Antrieb 35 bzw. 36 ausgehende Kraft auch auf das Plattenelement 7 zu übertragen, obwohl der entsprechende Antrieb 35 bzw. 36 nicht unterhalb des Plattenelements 7 angeordnet ist.

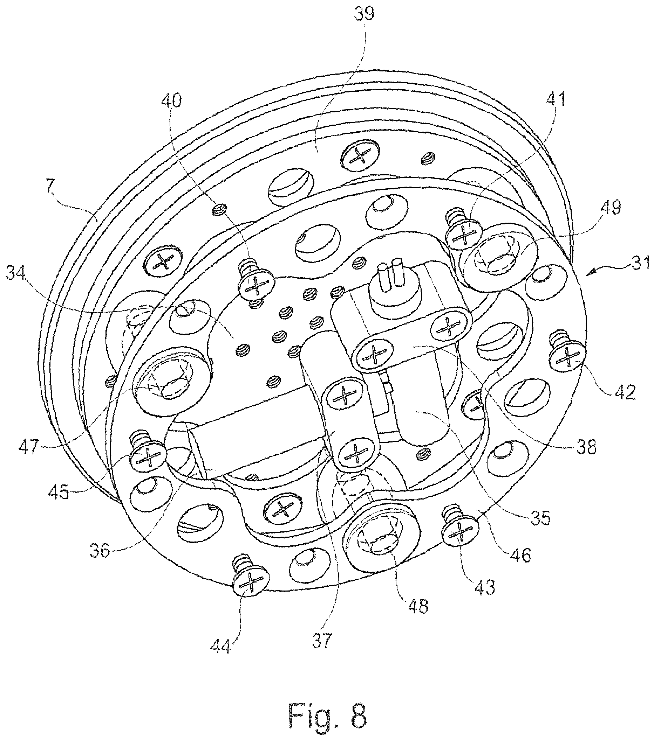

In Figur 8 ist eine erste Variante eines unteren Abschnitts des in Figur 7 gezeigten Plattenelements 7 dargestellt. Da der untere Abschnitt im Wesentlichen dem in Figur 7 gezeigten Abschnitt 31 entspricht, wurden die meisten Bezugszahlen beibehalten.

Im unteren Abschnitt 31 des Plattenelements 7 sind allerdings zwei Antriebe 35, 36 vorgesehen, die über Halteelemente 37, 38 mit dem in Figur 8 nicht dargestellten Halter verbunden sind. Diese beiden Antriebe 35, 36 können natürlich auch außerhalb des unteren Abschnitts 31 angeordnet sein. In diesem Fall könnte ein Kraftübertragungselement (nicht dargestellt) vorgesehen sein, das mit einem Ende mit dem entsprechenden Antrieb 35 bzw. 36 und mit dem anderen Ende mit dem entsprechenden Halteelement 37 bzw. 38 verbunden ist. Durch entsprechende Kraftübertragungselemente ist es möglich, eine von dem Antrieb 35 bzw. 36 ausgehende Kraft auch auf das Plattenelement 7 zu übertragen, obwohl der entsprechende Antrieb 35 bzw. 36 nicht unterhalb des Plattenelements 7 angeordnet ist.

Wie in Figur 8 zu erkennen, sind die Antriebe als Unwuchtmotoren ausgebildet, wobei es auch denkbar wäre, einen anderen Motorentyp, zum Beispiel einen Schrittmotor, als Antrieb einzusetzen. An der Unterseite 34 des Plattenelements 7 ist das erste Klemmelement 39 angeordnet, das über Verbindungsmittel 40 bis 45 mit dem zweiten Klemmelement 46 verbunden ist. Zwischen dem ersten Klemmelement 39 und dem zweiten Klemmelement 46 sind drei vertikal angeordnete Dämpfungselemente 47 bis 49 vorgesehen. Diese vertikal angeordneten Dämpfungselemente 47 bis 49 dienen dazu, die gerichteten Schwingungen, die durch die Antriebe 35, 36 erzeugt werden, in vertikaler Richtung zu dämpfen. Durch das Dämpfen der Schwingungen gleitet der auf dem Plattenelement 7 liegende Prüfling in die gewünschte Richtung, wohingegen der Prüfling ohne diese Dämpfungselemente 47 bis 49 auf dem Plattenelement 7 hüpfen könnte, womit die Bewegung des Prüflings unkontrolliert wäre. Die Dämpfungselemente 47 bis 49 können aus einem Elastomer bestehen, so beispielsweise aus Naturkautschuk oder aus Silikonkautschuk. Denkbar ist jedoch auch, dass die Dämpfungselemente 47 bis 49 aus einem Gummi-Metall-Dämpfer bestehen.

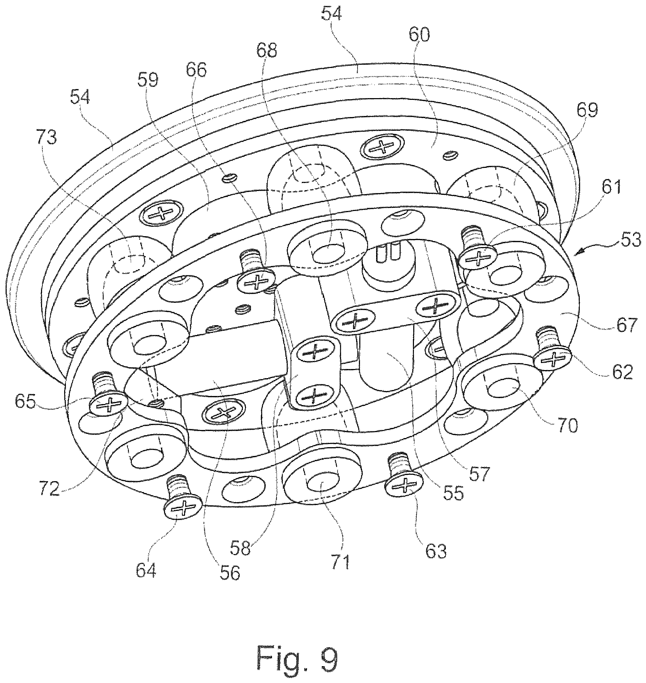

Figur 9 zeigt eine weitere Ausgestaltung eines unteren Abschnitts 53 eines Plattenelements 54. Im unteren Abschnitt 53 des Plattenelements 54 sind zwei Antriebe 55, 56 vorgesehen, die mittels eines entsprechenden Halteelements 57, 58 mit einem in Figur 9 nicht dargestellten Halter verbunden sind.

An einer Unterseite 59 des Plattenelements 7 ist ein erstes Klemmelement 60 vorgesehen, das über Verbindungsmittel 61 bis 66 mit einem zweiten Klemmelement 67 verbunden ist. Zwischen dem ersten Klemmelement 60 und dem zweiten Klemmelement 67 sind sechs vertikal angeordnete Dämpfungselemente 68 bis 73 vorgesehen. Anstelle der vertikal angeordneten Dämpfungselemente 68 bis 73 können auch Dämpfungselemente vorgesehen sein, die schräg zwischen den entsprechenden Klemmelementen angeordnet sind (nicht dargestellt). Wichtig bei diesen schräg angeordneten Dämpfungselementen ist lediglich, dass diese die Schwingung in vertikaler Richtung dämpfen.

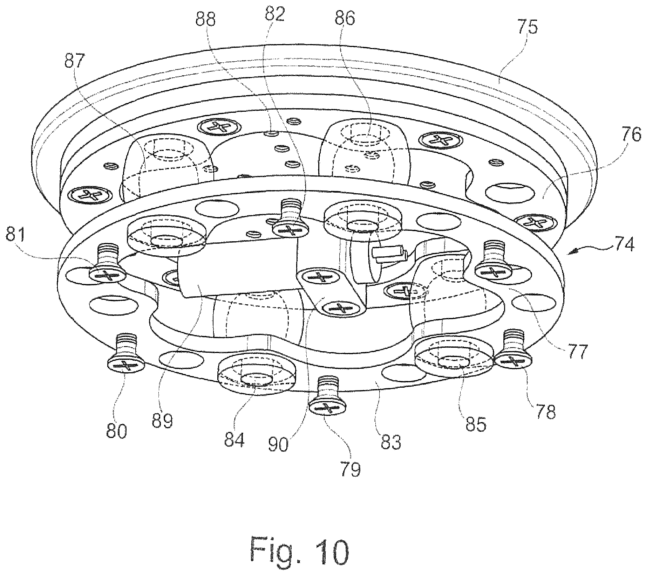

In Figur 10 ist eine weitere Ausgestaltung eines unteren Abschnitts 74 eines Plattenelements 75 dargestellt. Der untere Abschnitt 74 besteht aus einem ersten Klemmelement 76, das über Verbindungsmittel 77 bis 82 mit einem zweiten Klemmelement 83 verbunden ist. Die Klemmelemente 76 und 83 sind dabei als Klemmringe ausgebildet. Das Klemmelement 76 ist dabei an einer Unterseite 88 des Plattenelements 75 fixiert. Zwischen den beiden Klemmelementen 76 und 83 sind vier Dämpfungselemente 84 bis 87 angeordnet. Diese Dämpfungselemente 84 bis 87 können aus einem Elastomer bestehen. Möglich ist aber auch, dass die Dämpfungselemente 84 bis 87 ein Gummi-Metall-Dämpfer sind. In dem unteren Abschnitt 74 ist ein Antrieb 89 vorgesehen, der mittels eines Halteelements 90 an einem nicht weiter dargestellten Halter angebracht ist. Es versteht sich, dass diese Dämpfungselemente 84 bis 87 auch schräg zwischen den Klemmelementen angeordnet sein können. Diese schräg angeordneten Dämpfungselemente sind in diesem Fall derart zwischen den Klemmelementen angeordnet, dass diese die Schwingung ebenfalls in vertikaler Richtung dämpfen können. Diese Variante ist jedoch in der Figur 10 nicht gezeigt.

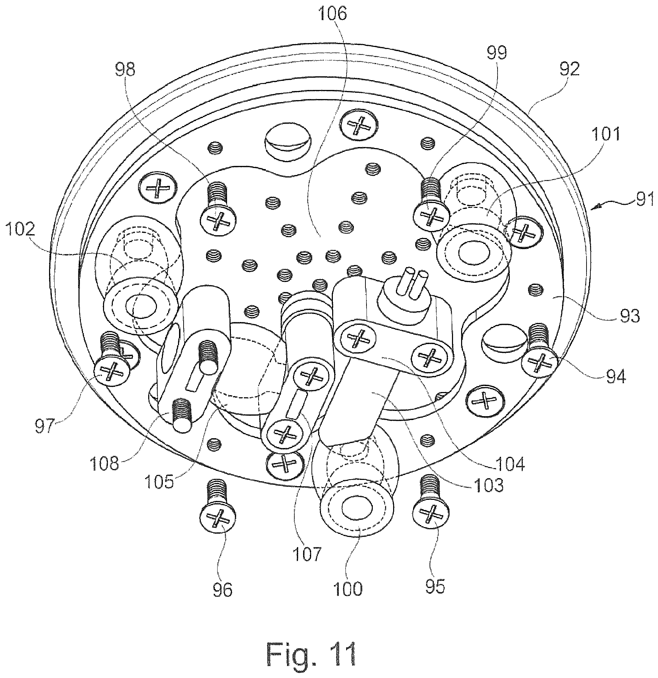

Figur 11 zeigt eine vierte Variante eines unteren Abschnitts 91 eines Plattenelements 92. Von diesem unteren Abschnitt 91 ist nur ein Klemmelement 93 dargestellt, das an einer Unterseite 107 des Plattenelements 92 fixiert ist. Das zweite Klemmelement, das über Verbindungsmittel 94 bis 99, zum Beispiel Schrauben, mit dem ersten Klemmelement 93 verbunden ist, ist Übersicht halber nicht gezeigt. Auch diese Klemmelemente sind als Klemmringe ausgebildet. Der untere Abschnitt 91 weist dabei dreivertikal angeordnete Dämpfungselemente 100 bis 102 auf. Im unteren Abschnitt 91 ist ein Antrieb 103 vorgesehen, der über ein Halteelement 104 mit einem in Figur 11 nicht dargestellten Halter verbunden ist. Durch diese vertikal angeordneten Dämpfungselemente 100 bis 102 wird die Vibration des Plattenelements 92 in vertikaler Richtung gedämpft. Ein Fachmann kann diese vertikal angeordneten Dämpfungselemente 100 bis 102 auch schräg zwischen den Klemmelementen anordnen, solange diese schräg angebrachten Dämpfungselemente die Schwingung in vertikaler Richtung dämpfen.

Im unteren Abschnitt 91 des Plattenelements 92 ist zusätzlich ein horizontal angeordnetes Dämpfungselement 105 vorgesehen, mit dem die Vibration in horizontaler Richtung gezielt abgebremst werden kann. Dieses horizontal angeordnete Dämpfungselement 105 ist über ein erstes Halteelement 107 mit der Unterseite 106 des Plattenelements 92 verbunden. Mit einem dem Halteelement 107 gegenüberliegenden Halteelement 108 ist das Dämpfungselement 105 mit dem in Figur 11 nicht dargestellten Halter verbunden. Auf diesem Halter ist der untere Abschnitt 91 entsprechend Figur 7 angeordnet. Es versteht sich, dass auch mehrere horizontal angeordnete Dämpfungselemente vorgesehen sein können.

Durch die horizontal angeordneten Dämpfungselemente 105 kann die Bewegung eines Prüflings auf dem Plattenelement 92 gezielt gesteuert werden, so dass der Prüfling beispielsweise nur in eine Richtung linear bewegt wird. Es versteht sich, dass auch mehr als nur ein horizontal angeordnetes Dämpfungselement vorgesehen werden kann.

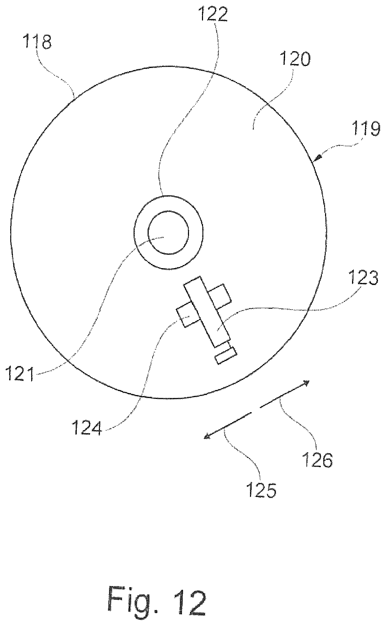

In Figur 12 ist eine fünfte Variante eines Plattenelements 118 mit einem unteren Abschnitt 119 gezeigt. Dabei wird direkt auf die Unterseite 120 des Plattenelements 118 geblickt. Bei dieser Variante handelt es sich um eine sehr einfache Variante, weil nur ein Dämpfungselement 121 vorgesehen ist. Dieses Dämpfungselement 121 ist an einem ersten Klemmelement 122 angeordnet. Das zweite Klemmelement, das gegenüber dem ersten Klemmelement angeordnet ist und zwischen denen das Dämpfungselement 121 angebracht ist, ist in Figur 12 nicht dargestellt. Das Dämpfungselement 121 kann beispielsweise ein Gummi-Metall-Dämpfer sein. Unterhalb des Plattenelements 118 ist ein Antrieb 123 vorgesehen, der über ein Halteelement 124 mit einem nicht dargestellten Halter verbunden ist. Bei diesem Antrieb 123 handelt es sich um einen Unwuchtmotor, mit dem es möglich ist, dass Plattenelement 118 in eine erste gerichtete Schwingung 125 sowie in eine ihr entgegengesetzte zweite gerichtete Schwingung 126 zu versetzen.

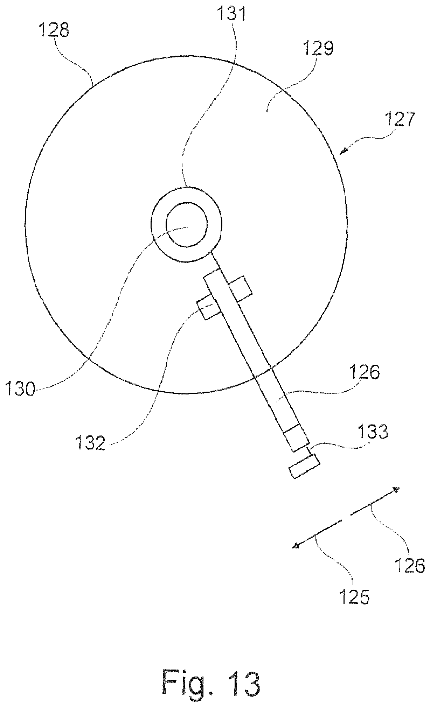

Figur 13 zeigt eine sechste Variante eines unteren Abschnitts 127 eines Plattenelements 128. Es wird direkt auf die Unterseite 129 des Plattenelements 128 geblickt. Wie auch die fünfte Variante besitzt dieses Plattenelement 128 ebenfalls nur ein Dämpfungselement 130, das an einem Klemmelement 131 angebracht ist. Auch dieses Dämpfungselement 130 ist im Zentrum der Unterseite 129 des Plattenelements 128 angeordnet. Bei diesem Dämpfungselement 130 handelt es sich vorzugsweise um einen Gummi-Metall-Dämpfer. Das zweite Klemmelement ist in dieser Figur der Übersicht halber nicht gezeigt. Das Plattenelement 128 ist ebenfalls mit einem Antrieb 133 verbunden. Auch bei diesem Antrieb 133 handelt es sich um einen Unwuchtmotor, der das Plattenelement 128 in eine erste sowie eine zweite gerichtete Schwingung versetzen kann, wobei die erste gerichtete Schwingung 134 der zweiten gerichteten Schwingungen 135 entgegengesetzt ist. Der Antrieb 133 sitzt allerdings nicht im unteren Abschnitt 127 und damit unterhalb des Plattenelements 128. Stattdessen ist der Antrieb 133 über eine Kraftübertragungselement 136 mit der Unterseite 129 des Plattenelements 128 verbunden. Dabei wird die Kraftübertragungselement 136 über eine Haltevorrichtung 132 an der Unterseite 129 des Plattenelements 128 gehalten.

Ein Fachmann kann natürlich die Dämpfungselemente 121 bzw. 130 gemäß den Figuren 12 und 13 auch schräg anordnen, solange gewährleistet ist, dass diese Dämpfungselemente die Schwingung in vertikaler Richtung dämpfen.

### Bezugszeichenliste

- 1: Bruchkammer
- 2: Prüfgerät
- 3: Festbacke
- 4: Pressbacke
- 5: Pfeil
- 6: Pfeil
- 7: Plattenelement
- 8: Boden
- 9: Widerlager
- 10: Prüfling
- 11: Verbindungselement
- 12: Verbindungselement
- 13: Transportvorrichtung
- 14: Prüfling
- 15: Prüfling
- 16: Pfeil
- 17: Transportebene
- 18: Öffnung
- 19: Abfallbehälter
- 20: Bruchkammer
- 21: Prüfgerät
- 22: Festbacke
- 23: Pressbacke
- 24: Pfeil
- 25: Pfeil
- 26: Plattenelement
- 27: Boden
- 28: Widerlager
- 29: Prüfling
- 30: Widerlager
- 31: Unterer Abschnitt
- 32: Gehäuse
- 33: Halter
- 34: Unterseite
- 35: Antrieb
- 36: Antrieb
- 37: Halteelement
- 38: Halteelement
- 39: Klemmelement
- 40: Verbindungsmittel
- 41: Verbindungsmittel
- 42: Verbindungsmittel
- 43: Verbindungsmittel
- 44: Verbindungsmittel
- 45: Verbindungsmittel
- 46: Klemmelement
- 47: Dämpfungselement
- 48: Dämpfungselement
- 49: Dämpfungselement
- 50: Antrieb
- 51: Halteelement
- 52: -
- 53: Unterer Abschnitt
- 54: Plattenelement
- 55: Antrieb
- 56: Antrieb
- 57: Halteelement
- 58: Halteelement
- 59: Unterseite
- 60: Klemmelement
- 61: Verbindungsmittel
- 62: Verbindungsmittel
- 63: Verbindungsmittel
- 64: Verbindungsmittel
- 65: Verbindungsmittel
- 66: Verbindungsmittel
- 67: Klemmelement
- 68: Dämpfungselement
- 69: Dämpfungselement
- 70: Dämpfungselement
- 71: Dämpfungselement
- 72: Dämpfungselement
- 73: Dämpfungselement
- 74: Unterer Abschnitt
- 75: Plattenelement
- 76: Klemmelement
- 77: Verbindungsmittel
- 78: Verbindungsmittel
- 79: Verbindungsmittel
- 80: Verbindungsmittel
- 81: Verbindungsmittel
- 82: Verbindungsmittel
- 83: Klemmelement
- 84: Dämpfungselement
- 85: Dämpfungselement
- 86: Dämpfungselement
- 87: Dämpfungselement
- 88: Unterseite
- 89: Antrieb
- 90: Halteelement
- 91: Unterer Abschnitt
- 92: Plattenelement
- 93: Klemmelement
- 94: Verbindungsmittel
- 95: Verbindungsmittel
- 96: Verbindungsmittel
- 97: Verbindungsmittel
- 98: Verbindungsmittel
- 99: Verbindungsmittel
- 100: Dämpfungselement
- 101: Dämpfungselement
- 102: Dämpfungselement
- 103: Antrieb
- 104: Halteelement
- 105: Dämpfungselement
- 106: Unterseite
- 107: Halteelement
- 108: Halteelement
- 109: Antrieb
- 110: Bewegungsrichtung
- 111: Bewegungsrichtung
- 112: -
- 113: Pfeil
- 114: Pfeil
- 115: Pfeil
- 116: Pfeil
- 117: Transportvorrichtung
- 118: Plattenelement
- 119: Unterer Abschnitt
- 120: Unterseite
- 121: Dämpfungselement
- 122: Klemmelement
- 123: Antrieb
- 124: Halteelement
- 125: Erste gerichtete Schwingung
- 126: Zweite gerichtete Schwingung
- 127: Unterer Abschnitt
- 128: Plattenelement
- 129: Unterseite
- 130: Dämpfungselement
- 131: Klemmelement
- 132: Haltevorrichtung
- 133: Antrieb
- 134: Erste gerichtete Schwingung
- 135: Zweite gerichtete Schwingung
- 136: Kraftübertragungselement

## Patentansprüche

1. Bruchkammer (1, 20) zum Messen einer Härte eines Prüflings (10, 29) umfassend eine Festbacke (3, 22) sowie eine ihr gegenüberliegende Pressbacke (4, 23), wobei die Bruchkammer (1, 20) einen Boden (8, 27) aufweist, wobei ein erstes Widerlager (9, 28) vorgesehen ist, das die Bruchkammer (1, 20) zu einer Seite hin begrenzt und sich von der Festbacke (3, 22) in Richtung der Pressbacke (4, 23) erstreckt, wobei das Widerlager (9, 28) im Wesentlichen in einem rechten Winkel zu der Festbacke (3, 22) sowie zu der Pressbacke (4, 23) angeordnet ist, wobei der Boden (8, 27) durch mindestens einen Teil eines Plattenelements (7, 26, 54, 75, 92, 128, 118) gebildet wird, **dadurch gekennzeichnet, dass** mindestens ein Antrieb (35, 36, 50, 55, 56, 89, 103, 123, 133) vorgesehen ist, der mit dem Plattenelement (7, 26, 54, 75, 92, 128, 118) verbunden ist, wobei das Plattenelement (7, 26, 54, 75, 92, 128, 118) mit einem ersten Antrieb in eine erste gerichtete Schwingung und nach Umpolung des ersten Antriebs (50, 89, 103, 123, 133) in eine zweite gerichtete Schwingung versetzbar ist oder wobei das Plattenelement (7, 26, 54, 75, 92, 128, 118) durch Einschalten eines ersten Antriebs in eine erste gerichtete Schwingung und durch Abschalten des ersten Antriebs und Inbetriebnahme eines zweiten Antriebs in eine zweite gerichtete Schwingung versetzbar ist, wobei die erste gerichtete Schwingung entgegengesetzt zu der zweiten gerichteten Schwingung ist, wobei der auf dem Plattenelement (7, 26, 75, 92, 128, 118) liegende Prüfling (10, 29) durch die erste gerichtete Schwingung in eine erste Bewegungsrichtung (110) und durch in die zweite gerichtete Schwingung in eine zweite Bewegungsrichtung (111) bewegbar ist, wobei die erste Bewegungsrichtung (110) des Prüflings (10, 29) der zweiten Bewegungsrichtung (111) entgegengesetzt ist, wodurch der Prüfling (10, 29) in der Bruchkammer (1, 20) positionierbar ist.

2. Bruchkammer nach Patentanspruch 1, **dadurch gekennzeichnet, dass** der Antrieb (35, 36, 55, 56, 89, 103, 123, 133) ein Unwuchtmotor ist.

3. Bruchkammer nach Patentanspruch 1, **dadurch gekennzeichnet, dass** unterhalb des Plattenelements (7, 26, 54, 75, 92, 128, 118) mindestens ein angeordnetes Dämpfungselement (47 bis 49, 68 bis 73, 84 bis 87, 100 bis 102, 105, 121, 130) vorgesehen ist, das zwischen einem ersten Klemmelement (39, 60, 76, 93, 122, 131) und einem zweiten Klemmelement (46, 67, 83) angeordnet ist, wobei das erste Klemmelement (39, 60, 76, 93, 122, 131) mit dem Plattenelement (39, 60, 76, 93, 128, 118) verbunden ist.

4. Bruchkammer nach Patentanspruch 3, **dadurch gekennzeichnet, dass** zwischen dem ersten Klemmelement (39, 60, 76, 93) und dem zweiten Klemmelement (46, 67, 83) mindestens drei Dämpfungselemente angeordnet sind.

5. Bruchkammer nach Patentanspruch 3 oder 4, **dadurch gekennzeichnet, dass** das mindestens eine Dämpfungselement (47 bis 49, 68 bis 73, 84 bis 87, 100 bis 102, 105, 121, 130) ein Elastomer enthält.

6. Bruchkammer nach Patentanspruch 3 oder 4, **dadurch gekennzeichnet, dass** das mindestens eine Dämpfungselement (47 bis 49, 68 bis 73, 84 bis 87, 100 bis 102, 105, 121, 130) ein Gummi-Metall-Dämpfer ist.

7. Verfahren zum Durchführen einer Härtemessung an einem Prüfling in einer Bruchkammer nach einem der Patentansprüche 1 bis 6, **dadurch gekennzeichnet, dass** das Verfahren folgende aufeinanderfolgende Schritte umfasst:
1. Es wird ein Prüfling in der Bruchkammer bereitgestellt, wobei der Prüfling mit einer ersten Längsseite an dem ersten Widerlager anliegt;
2. es wird die Pressbacke in Richtung der Festbacke bewegt, bis der Prüfling zwischen der Pressbacke und der Festbacke eingeklemmt ist, wobei ein Antrieb eingeschaltet wird, wodurch das Plattenelement in eine zweite gerichtete Schwingung versetzt wird, wobei der Prüfling durch die zweite gerichtete Schwingung an dem ersten Widerlager gehalten wird;
3. es wird die Länge des Prüflings vermessen;
4. die Pressbacke wird weiter in Richtung der Festbacke bewegt und es wird so lange Kraft auf den Prüfling ausgeübt, bis dieser zerbricht, wobei zwischen den Schritten 3 und 4 folgende aufeinanderfolgende Schritte folgen:
1. die Pressbacke wird in eine Ausgangsposition zurückgefahren;
2. ein Antrieb wird eingeschaltet und das Plattenelement dadurch in eine erste gerichtete Schwingung versetzt, wodurch der Prüfling gedreht wird, bis der Prüfling mit seiner zweiten Längsseite an der Festbacke anliegt;
3. die Pressbacke wird in Richtung der Festbacke geschoben, bis der Prüfling zwischen der Festbacke und der Pressbacke eingeklemmt ist;
4. es wird die Breite des Prüflings vermessen;
5. die Pressbacke wird in ihre Ausgangsposition zurückgefahren;
6. der Antrieb wird umgepolt und das Plattenelement in die zweite gerichtete Schwingung versetzt, wodurch der Prüfling gedreht wird, so dass dieser Prüfling mit seiner ersten Längsseite wieder an dem Widerlager angeordnet ist.

8. Verfahren nach Patentanspruch 7, **dadurch gekennzeichnet, dass** das Verfahren zwischen den Schritten 1 und 2 folgende aufeinanderfolgende Schritte aufweist:
1. ein Antrieb wird eingeschaltet und das Plattenelement in eine erste gerichtete Schwingung versetzt, wodurch der Prüfling in der Bruchkammer derart umpositioniert wird, dass der Winkel α der ersten Längsseite in Bezug auf das erste Widerlager etwa 35° bis 55° und vorzugsweise etwa 45° beträgt;
2. die Pressbacke wird in Richtung der Festbacke bewegt, bis der Prüfling mit seiner zweiten Längsseite an der Festbacke anliegt und zwischen der Festbacke und der Pressbacke eingeklemmt ist;
3. es wird die Breite des Prüflings vermessen;
4. die Pressbacke wird wieder in die Ausgangsposition zurückbewegt;
5. der Antrieb versetzt das Plattenelement in eine zweite gerichtete Schwingung, wodurch der Prüfung in der Bruchkammer derart umpositioniert wird, dass der Prüfung mit seiner ersten Längsseite wieder an dem Widerlager anliegt.

9. Verfahren zum Durchführen einer Härtemessung an einem Prüfling in einer Bruchkammer nach einem der Patentansprüche 1 bis 6, **dadurch gekennzeichnet, dass** das Verfahren folgende aufeinanderfolgende Schritte umfasst:
1. Es wird ein Prüfling in der Bruchkammer bereitgestellt, wobei der Prüfling im Wesentlichen parallel zu einer schrägen Seite des Widerlagers angeordnet ist;
2. die Pressbacke wird in Richtung der Festbacke bewegt, bis der Prüfling mit einer ersten Längsseite an der Pressbacke anliegt, wobei der Prüfling zudem zwischen der Pressbacke und der Festbacke eingeklemmt ist;
3. es wird die Breite des Prüflings vermessen;
4. die Pressbacke wird in eine Ausgangsposition zurückgefahren;
5. der Antrieb wird eingeschaltet und das Plattenelement in eine erste gerichtete Schwingung versetzt, wodurch der Prüfling gedreht wird, bis der Prüfling mit seiner zweiten Längsseite an der Pressbacke angeordnet ist;
6. die Pressbacke wird in Richtung der Festbacke bewegt, bis der Prüfling zwischen der Festbacke und der Pressbacke eingeklemmt ist;
7. es wird die Länge des Prüflings vermessen;
8. die Pressbacke wird weiter in Richtung der Festbacke bewegt und es wird so lange Kraft auf den Prüfling ausgeübt, bis dieser zerbricht.

## Claims

1. Crush Chamber (1, 20) for measuring the hardness of a test sample (10, 29), comprising a fixed jaw (3, 22) as well as a pressing jaw (4, 23) opposite thereto, wherein the crush chamber (1, 20) comprises a bottom (8, 27), wherein a first abutment (9, 28) is provided that delimits the crush chamber (1, 20) toward one side and extends from the fixed jaw (3, 22) in the direction toward the pressing jaw (4, 23) with the abutment (9, 28) being substantially disposed at a right angle to the fixed jaw (3, 22) as well as to the pressing jaw (4, 23), wherein the bottom (8, 27) is formed by at least a portion of a plate element (7, 26, 54, 75, 92, 128, 118) **characterized in that** at least one drive (35, 36, 50, 55, 56, 89, 103, 123, 133) is provided that is in connection with the plate element (7, 26, 54, 75, 92, 128, 118), wherein the plate element (7, 26, 54, 75, 92, 128, 118) can be set into a first directed vibration with a first drive and, after reversal of the polarity of the first drive (50, 89, 103, 123, 133) can be set into a second directed vibration or wherein the plate element (7, 26, 54, 75, 92, 128, 118) can be set into a first directed vibration by switching on the first drive [50, 89,103, 123, 133] and, by switching off and putting into operation a second drive, can be set into a second directed vibration, wherein the first directed vibration is opposite to the second directed vibration, wherein the test sample (10, 29) located on the plate element (7, 26, 75, 92, 128, 118) is movable through the first directed vibration into a first direction of motion (110) and through the second directed vibration into a second direction of motion (111), with the first direction of motion (110) of the test sample (10, 29) being opposite to the second direction of motion (111), whereby the test sample (10, 29) is positionable in the crush chamber (1, 20).

2. Crush chamber as in patent claim 1, **characterized in that** the drive (35, 36, 55, 56, 89, 103, 123, 133) is an unbalance motor.

3. Crush chamber as in patent claim 1, **characterized in that** at least one damping element (47 to 49, 68 to 73, 84 to 87, 100 to 102, 105, 121, 130) is provided that is disposed underneath the plate element (7, 26, 54, 75, 92, 128, 118) and between a first clamping element (39, 60, 76, 93, 122, 131) and a second clamping element (46, 67, 83), wherein the first clamping element (39, 60, 76, 93, 122, 131) is connected with the plate element (39, 60, 76, 93, 122, 131) [*sic:* 7, 26, 54, 75, 92, 128, 118].

4. Crush chamber as in patent claim 3, **characterized in that** between the first clamping element (39, 60, 76, 93) and the second clamping element (46, 67, 83) at least three damping elements are disposed.

5. Crush chamber as in patent claim 3 or 4, **characterized in that** at least one damping element (47 to 49, 68 to 73, 84 to 87, 100 to 102, 105, 121, 130) comprises an elastomer.

6. Crush chamber as in patent claim 3 or 4, **characterized in that** at least one damping element (47 to 49, 68 to 73, 84 to 87, 100 to 102, 105, 121, 130) is a rubber metal damper.

7. Method for carrying out a hardness measurement of a test sample in a crush chamber as in one of patent claims 1 to 6, **characterized in that** the method comprises the following sequential steps:
1. a test sample is positioned in the crush chamber with the test sample being in contact with a first longitudinal side on the first abutment;
2. the pressing jaw is moved in the direction toward the fixed jaw until the test sample is clamped between the pressing jaw and the fixed jaw, wherein a drive is switched on whereby the plate element is set into a second directed vibration with the test sample being held in contact on the first abutment through the second directed vibration;
3. the length of the test sample is measured;
4. the pressing jaw is moved further in the direction toward the fixed jaw and force is exerted onto the test sample until it fractures, wherein between steps 3 and 4 the following sequential steps are completed:
1. the pressing jaw is retracted into a starting position;
2. a drive is switched on and the plate element is thereby set into a first directed vibration, whereby the test sample is rotated until the test sample is in contact with its second longitudinal side on the fixed jaw;
3. the pressing jaw is moved in the direction toward the fixed jaw until the test sample is clamped between the fixed jaw and the pressing jaw;
4. the width of the test sample is measured;
5. the pressing jaw is retracted into its starting position;
6. the polarity of the drive is reversed and the plate element is set into the second
directed vibration whereby the test sample is rotated such that this test sample is again disposed with its first longitudinal side on the abutment.

8. Method as in patent claim 7, **characterized in that** the method comprises the following sequential steps between step 1 and 2:
1. a drive is switched on and the plate element is set into a first directed vibration whereby the test sample in the crush chamber is repositioned such that the angle α of the first longitudinal side with respect to the first abutment is approximately 35° to 55° and preferably approximately 45°;
2. the pressing jaw is moved in the direction toward the fixed jaw until the test sample is in contact with its second longitudinal side on the fixed jaw and is clamped between the fixed jaw and the pressing jaw;
3. the width of the test sample is measured;
4. the pressing jaw is again retracted into the starting position;
5. the drive sets the plate element into a second directed vibration, whereby the test sample in the crush chamber is repositioned such that the test sample is again in contact with its first longitudinal side on the abutment.

9. Method for carrying out a hardness measurement of a test sample in a crush chamber as in one of patent claims 1 to 6, **characterized in that** the method comprises the following sequential steps:
1. a test sample is positioned in the crush chamber wherein the test sample is essentially disposed parallel to an oblique side of the abutment;
2. the pressing jaw is moved in the direction toward the fixed jaw until the test sample is in contact with a first longitudinal side on the pressing jaw whereby the test sample is moreover clamped between the pressing jaw and the fixed jaw;
3. the width of the test sample is measured;
4. the pressing jaw is retracted into a starting position;
5. the drive is switched on and the plate element is set into a first directed vibration whereby the test sample is rotated until the test sample is disposed with its second longitudinal side on the pressing jaw;
6. the pressing jaw is moved in the direction toward the fixed jaw until the test sample is clamped between the fixed jaw and the pressing jaw;
7. the length of the test sample is measured;
8. the pressing jaw is moved further in the direction toward the fixed jaw and force is exerted onto the test sample until it fractures.

## Revendications

1. Chambre de rupture (1, 20) permettant de mesurer une dureté d'une éprouvette (10, 29), comprenant une mâchoire fixe (3, 22) et une mâchoire de pression (4, 23) qui lui fait face, dans laquelle la chambre de rupture (1, 20) présente un fond (8, 27), dans laquelle il est prévu une première butée (9, 28) qui délimite la chambre de rupture (1, 20) d'un côté et qui s'étend depuis la mâchoire fixe (3, 22) dans la direction de la mâchoire de pression (4, 23), dans laquelle la butée (9, 28) est disposée sensiblement à angle droit par rapport à la mâchoire fixe (3, 22) et à la mâchoire de pression (4, 23), dans laquelle le fond (8, 27) est formé par au moins une partie d'un élément en forme de plaque (7, 26, 54, 75, 92, 128, 118), **caractérisé en ce qu'**il est prévu au moins un système d'entraînement (35, 36, 50, 55, 56, 89, 103, 123, 133) qui est relié à l'élément en forme de plaque (7, 26, 54, 54, 75, 92, 128, 118), dans laquelle l'élément en forme de plaque (7, 26, 54, 75, 92, 128, 118) peut être soumis à une première oscillation dirigée au moyen d'un premier système d'entraînement et, après inversion de polarité du premier système d'entraînement (50, 89, 103, 123, 133), à une seconde oscillation dirigée ou dans laquelle l'élément en forme de plaque (7, 26, 54, 75, 92, 128, 118) peut être soumis à une première oscillation dirigée au moyen en allumant du premier système d'entraînement et à une seconde oscillation dirigée au moyen en éteignant du première system d'entraînement et par la mise en service d'un second système d'entraînement, dans laquelle la première oscillation est opposée directionelle, dans laquelle l'éprouvette (10, 29), qui repose sur l'élément en forme de plaque (7, 26, 75, 25, 92, 128, 118), peut être déplacée par la première oscillation dirigée dans une première direction de déplacement (110) et par la seconde oscillation dirigée dans une seconde direction de déplacement (111), dans laquelle la première direction de déplacement (110) de l'éprouvette (10, 29) est opposée à la seconde direction de déplacement (111), cela permettant de positionner l'éprouvette (10, 29) dans la chambre de rupture (1, 20).

2. Chambre de rupture selon la revendication 1, **caractérisée en ce que** le système d'entraînement (35, 36, 55, 56, 89, 103, 123, 133) est un moteur à balourds.

3. Chambre de rupture selon la revendication 1, **caractérisée en ce qu'**il est prévu en-dessous de l'élément en forme de plaque (7, 26, 54, 75, 92, 128, 118) au moins un élément amortisseur (47 à 49, 68 à 73, 84 à 87, 100 à 102, 105, 121, 130) qui est disposé entre un premier élément de serrage (39, 60, 76, 93, 122, 131) et un second élément de serrage (46, 67, 83), dans laquelle le premier élément de serrage (39, 60, 76, 93, 122, 131) est relié à l'élément en forme de plaque (39, 60, 76, 93, 128, 118).

4. Chambre de rupture selon la revendication 3, **caractérisée en ce qu'**au moins trois éléments amortisseurs sont disposés entre le premier élément de serrage (39, 60, 76, 93) et le second élément de serrage (46, 67, 83).

5. Chambre de rupture selon la revendication 3 ou 4, **caractérisée en ce que** ledit au moins un élément amortisseur (47 à 49, 68 à 73, 84 à 87, 100 à 102, 105, 121, 130) contient un élastomère.

6. Chambre de rupture selon la revendication 3 ou 4, **caractérisée en ce que** ledit au moins un élément amortisseur (47 à 49, 68 à 73, 84 à 87, 100 à 102, 105, 121, 130) est un amortisseur en caoutchouc-métal.

7. Procédé permettant d'effectuer une mesure de dureté sur une éprouvette dans une chambre de rupture selon l'une des revendications 1 à 6, **caractérisé en ce que** le procédé comprend les étapes successives suivantes :
1. une éprouvette est fournie dans la chambre de rupture, dans lequel le premier côté longitudinal de l'éprouvette repose sur la première butée ;
2. la mâchoire de pression est déplacée dans la direction de la mâchoire fixe jusqu'à ce que l'éprouvette soit serrée entre la mâchoire de pression et la mâchoire fixe, dans lequel un système d'entraînement est mis en marche, de sorte que l'élément en forme de plaque est soumis à une seconde oscillation dirigée, dans lequel l'éprouvette est maintenue sur la première butée par la seconde oscillation dirigée ;
3. la longueur de l'éprouvette est mesurée ;
4. la mâchoire de pression est déplacée davantage dans la direction de la mâchoire fixe et une force est appliquée à l'éprouvette jusqu'à ce qu'elle se brise, dans lequel les étapes 3 et 4 sont suivies des étapes successives suivantes :
1. la mâchoire de pression est ramenée à une position initiale ;
2. un système d'entraînement est mis en marche et l'élément en forme de plaque est ainsi soumis à une première oscillation dirigée, de sorte que l'éprouvette est mise en rotation jusqu'à ce que son second côté longitudinal repose contre la mâchoire fixe ;
3. la mâchoire de pression est repoussée dans la direction de la mâchoire fixe jusqu'à ce que l'éprouvette soit serrée entre la mâchoire fixe et la mâchoire de pression ;
4. la largeur de l'éprouvette est mesurée ;
5. la mâchoire de pression est ramenée à sa position initiale ;
6. le système d'entraînement est soumis à une inversion de polarité et l'élément en forme de plaque est soumis à la seconde oscillation dirigée, de sorte que l'éprouvette est mise en rotation de manière à ce que le premier côté longitudinal de ladite éprouvette soit de nouveau disposé contre la butée.

8. Procédé selon la revendication 7, **caractérisé en ce que** le procédé comprend les étapes successives suivantes entre les étapes 1 et 2 :
1. un système d'entraînement est mis en marche et l'élément en forme de plaque est soumis à une première oscillation dirigée, de sorte que l'éprouvette est repositionnée dans la chambre de rupture de manière à ce que l'angle □ du premier côté longitudinal par rapport à la première butée soit d'environ 35° à 55° et de préférence d'environ 45° ;
2. la mâchoire de pression est déplacée dans la direction de la mâchoire fixe jusqu'à ce que le second côté longitudinal de l'éprouvette repose contre la mâchoire fixe et soit serré entre la mâchoire fixe et la mâchoire de pression ;
3. la largeur de l'éprouvette est mesurée ;
4. la mâchoire de pression est ramenée à la position initiale ;
5. le système d'entraînement soumet l'élément en forme de plaque à une seconde oscillation dirigée, de sorte que l'éprouvette est repositionnée dans la chambre de rupture de manière à ce que le premier côté longitudinal de l'éprouvette repose contre la butée.

9. Procédé permettant d'effectuer une mesure de dureté sur une éprouvette dans une chambre de rupture selon l'une des revendications 1 à 6, **caractérisé en ce que** le procédé comprend les étapes successives suivantes :
1. une éprouvette est fournie dans la chambre de rupture, dans lequel l'éprouvette est disposée sensiblement parallèlement à une face inclinée de la butée ;
2. la mâchoire de pression est déplacée dans la direction de la mâchoire fixe jusqu'à ce qu'un premier côté longitudinal de l'éprouvette repose contre la mâchoire de pression, dans lequel l'éprouvette est en outre serrée entre la mâchoire de pression et la mâchoire fixe ;
3. la largeur de l'éprouvette est mesurée ;
4. la mâchoire de pression est ramenée à une position initiale ;
5. le système d'entraînement est mis en marche et l'élément en forme de plaque est soumis à une première oscillation dirigée, de sorte que l'éprouvette est mise en rotation jusqu'à ce que le second côté longitudinal de l'éprouvette soit disposé contre la mâchoire de pression ;
6. la mâchoire de pression est déplacée dans la direction de la mâchoire fixe jusqu'à ce que l'éprouvette soit serrée entre la mâchoire fixe et la mâchoire de pression ;
7. la longueur de l'éprouvette est mesurée ;
8. la mâchoire de pression est déplacée davantage dans la direction de la mâchoire fixe et une force est appliquée à l'éprouvette jusqu'à ce qu'elle se brise.
